(19) Europäisches Patentamt
European Patent Office
Office européen des brevets

(11) Veröffentlichungsnummer: **0 268 775 B1**

(12) **EUROPÄISCHE PATENTSCHRIFT**

(45) Veröffentlichungstag der Patentschrift: **23.12.92**

(51) Int. Cl.[5]: **C07D 213/79**, C07D 213/83, A01N 43/40, C07D 265/30, A01N 43/84

(21) Anmeldenummer: **87113916.8**

(22) Anmeldetag: **23.09.87**

# (54) Verfahren zum Schutz von Pflanzen gegen Krankheiten.

(30) Priorität: **26.09.86 CH 3866/86**
**17.07.87 CH 2730/87**

(43) Veröffentlichungstag der Anmeldung:
**01.06.88 Patentblatt 88/22**

(45) Bekanntmachung des Hinweises auf die Patenterteilung:
**23.12.92 Patentblatt 92/52**

(84) Benannte Vertragsstaaten:
**AT BE CH DE ES FR GB GR IT LI LU NL**

(56) Entgegenhaltungen:
**CH-A- 302 905**
**DE-C- 1 135 238**
**GB-A- 1 334 036**
**US-A- 4 137 067**
**US-A- 4 203 988**

**BEILSTEINS HANDBUCH DER ORGANISCHEN CHEMIE, 4. Auflage, Band 22, 1935, Seite 48, Julius Springer Verlag, Berlin**

(73) Patentinhaber: **CIBA-GEIGY AG**
**Klybeckstrasse 141**
**CH-4002 Basel(CH)**

(72) Erfinder: **Kunz, Walter, Dr.**
**Buchenstrasse 9**
**CH-4104 Oberwil(CH)**
Erfinder: **Staub, Theodor, Dr.**
**Rheintalweg 90**
**CH-4125 Riehen(CH)**
Erfinder: **Métraux, Jean-Pierre, Dr.**
**Bruderholzallee 166**
**CH-4059 Basel(CH)**
Erfinder: **Hoegerle, Karl, Dr.**
**Conrad Ferdinand Meyer-Strasse 54**
**CH-4059 Basel(CH)**
Erfinder: **Nyfeler, Robert, Dr.**
**Bärenfelserstrasse 8**
**CH-4057 Basel(CH)**
Erfinder: **Ahl, Patricia A., Dr.**
**St. Johannsring 109**
**CH-4056 Basel(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**CHEMICAL ABSTRACTS, Band 57, Nr. 4, 20. August 1962, Spalte 4769 a-c, Columbus, Ohio, US; K. PALAT et al.: "Tuberculostatic substances from derivatives of pyridine carboxylic acids"**

**PESTICIDE SCIENCE, Band 15, Nr. 3, Juni 1984, Seiten 285-295; E.H. POMMER "Chemical structure-fungicidal activity relationships in substituted morpholines"**

(74) Vertreter: **Zumstein, Fritz, Dr. et al**
**Bräuhausstrasse 4**
**W-8000 München 2(DE)**

## Beschreibung

Die vorliegende Erfindung betrifft ein Verfahren zur Immunisierung von gesunden Pflanzen gegen Pflanzenkrankheiten, vor allem gegen den Befall von Pflanzen durch phytopathogene Mikroorganismen, insbesondere durch Fungi. Das erfindungsgemässe Verfahren besteht darin, dass Wirkstoffe aus Pflanzen oder deren Umgebung appliziert werden. Die eingesetzten Wirkstoffe sind Verbindungen folgender allgemeiner Formel I:

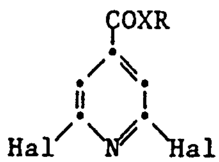

(I)

in welcher

Hal für Halogen steht,

X Sauerstoff oder Schwefel bedeutet und

R Wasserstoff, $C_1$-$C_6$-Alkyl, durch ein Sauerstoff- oder Schwefelatom unterbrochenes $C_1$-$C_6$-Alkyl substituiertes $C_1$-$C_6$-Alkyl, unsubstituiertes $C_3$-$C_5$-Alkenyl oder durch Halogen substituiertes $C_3$-$C_5$-Alkenyl, unsubstituiertes $C_3$-$C_5$-Alkinyl, unsubstituiertes $C_3$-$C_6$-Cyclolkyl oder durch Halogen oder Methyl substituiertes $C_3$-$C_6$-Cycloalkyl, oder ein Normaläquivalent eines Kations darstellt, das aus einer Base oder einer basischen Verbindung gebildet ist.

Halogen selbst oder als Bestandteil eines anderen Substituenten bedeutet Fluor, Chlor, Brom oder Jod, vorzugsweise Chlor, Brom oder Jod.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl, Butyl, Pentyl oder Hexyl, wie z.B. Isopropyl, Isobutyl, tert.-Butyl, sek.-Butyl oder Isopentyl. Cycloalkyl bedeutet wahlweise Cyclopropyl, Cyclobutyl, Cyclopentyl oder Cyclohexyl.

Alkenyl bedeutet z.B. Propenyl-(1), Allyl, Butenyl-(1), Butenyl-(2) oder Butenyl-(3) sowie Ketten mit mehreren Doppelbindungen. Alkinyl bedeutet z.B. Propinyl-(2), Butinyl-(1), Butinyl-(2), Pentinyl-(4) usw., vorzugsweise Propargyl.

Als Basen oder Verbindungen basischen Charakters kommen anorganische oder organische Basen oder Basenbildner in Betracht. So sind z.B. unter anorganischen Basen Hydroxide, Carbonate und Hydrogencarbonate von Alkali- und Erdalkalimetallen, vorzugsweise LiOH, NaOH, KOH, $Mg(OH)_2$ oder $Ca(OH)_2$; und ferner $NaHCO_3$, $KHCO_3$, $Na_2CO_3$ und $K_2CO_3$ zu verstehen. Als organische Basen gelten aliphatische Alkylamine mit 1 bis 3 ($C_1$-$C_6$)-Alkylgruppen, die gegebenenfalls durch ein Sauerstoffatom oder mehrere Sauerstoffatome unterbrochen sind. Bevorzugt sind darunter Alkylamine mit $C_1$-$C_3$-Alkylgruppen, z.B. tertiäre Amine wie Trimethylamin, Triethylamin oder Tripropylamin, sowie z.B. das Amin $N(C_2H_4-OC_2H_5)_2CH_3$. Unter organischen Basen sind ferner cyclische Alkylamine zu verstehen, die von folgenden Verbindungen repräsentiert werden: heterocyclischen Amine vom Morpholintyp der Formeln $A_1$ und $A_2$

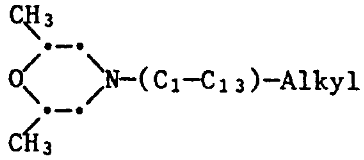

($A_1$)

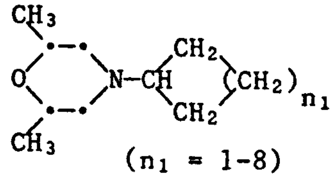

($A_2$)

wie z.B.

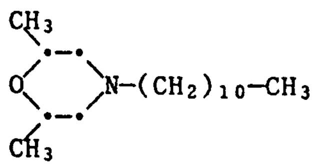

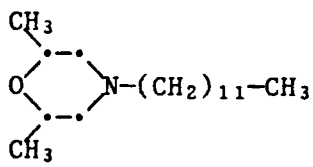

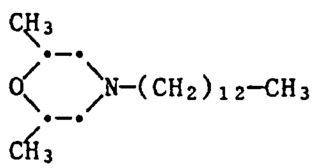

wie z.B.

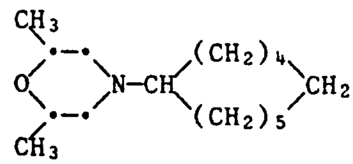

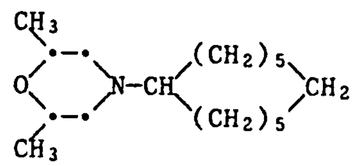

oder vom Typ der Formel B

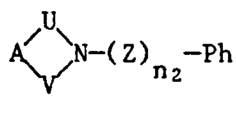 (B)

worin

A Sauerstoff oder die Methylengruppe;

U und V $C_1$-$C_3$-Alkylen oder $C_1$-$C_3$-Alkylalkylen, vorzugsweise Ethylen oder durch Methyl substituiertes Ethylen;

Z $C_1$-$C_4$-Alkylen, z.B. die Gruppe

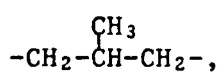

und

Ph unsubstituiertes oder durch $C_1$-$C_4$-Alkyl, vorzugsweise 4-tert.-Butyl, substituiertes Phenyl darstellen und $n_2$ = 0 oder 1 bedeutet,

darunter z.B. insbesondere die Verbindung $B_1$

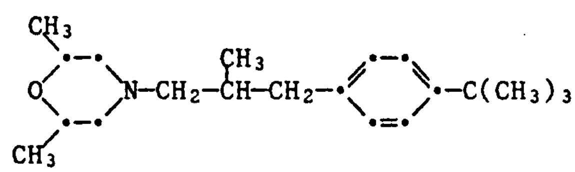 ($B_1$)

oder die Verbindung $B_2$

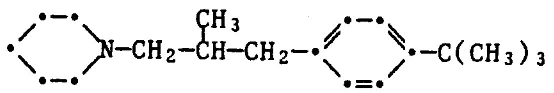

(B$_2$),

wobei die aufgrund von chiralen Strukturen der kationischen Verbindungen der Formeln (A) und (B) sich ergebenden Enantiomeren miteingeschlossen sind.

Speziell ist diejenige Konfiguration der Verbindung B$_1$ bevorzugt, in der die beiden Methylgruppen am Morpholin in cis-Stellung zueinander stehen. Von den beiden enantiomeren cis-Formen ist ferner die (-)-Konfiguration besonders bevorzugt. Die cis-Konfiguration der Formel B$_1$ ist durch folgende Formel darstellbar:

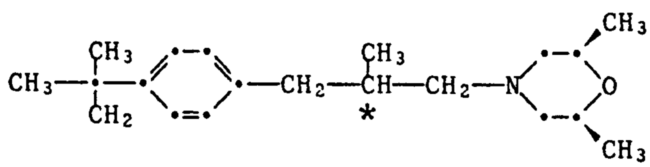

Aufgrund ihrer ausgeprägten gegen Fungibefall immunisierenden Wirksamkeit sind diejenigen Wirksubstanzen bevorzugt, die folgende Substituenten oder Kombinationen dieser Substituenten untereinander aufweisen:

1) X steht für Sauerstoff;

a) Hal bedeutet gemäss nachfolgender Rangordnung der Bevorzugung: Chlor, Brom, Jod oder Fluor, insbesonder Chlor oder Brom, wobei die Substitution der 2- und 6-Stellung mit gleichartigen Halogenatomen bevorzugt ist, und R bedeutet Wasserstoff, Methyl, Ethyl, n-Propyl, iso-Propyl oder n-Butyl oder als Normaläquivalent eines Kations: Natrium, 4-[3-(4-tert.Butylphenyl)-2-methylprop-1-yl]-2,6-dimethylmorpholin oder N-[3-(4-tert.Butylphenyl)-2-methylprop-1-yl]-piperidin oder 4-Cyclodecyl-2,6-dimethylmorpholin;

b) Hal bedeutet 2,6-Dichlor, 2,6-Dibrom oder 2,6-Dijod und R bedeutet Wasserstoff, Methyl oder Ethyl oder als Normaläquivalent eines Kations: 4-[3-(4-tert.Butylphenyl)-2-methylprop-1-yl]-2,6-dimethylmorpholin oder N-[3-(4-tert.Butylphenyl)-2-methylprop-1-yl]-pyrimidin.

2) X steht für Schwefel;

a) Hal bedeutet gemäss nachfolgender Rangordnung der Bevorzugung: Chlor, Brom, Jod oder Fluor, insbesondere Chlor oder Brom, wobei die Substitution der 2- und 6-Stellung mit gleichartigen Halogenatomen bevorzugt ist, und R bedeutet Wasserstoff, Methyl oder Ethyl oder als Normaläquiv6lent eines Kations: Natrium 4-[3-(4-tert.Butylphenyl)-2-methylprop-1-yl]-2,6-dimethylmorpholin oder N-[3-(4-tert.Butylphenyl)-2-methylprop-1-yl]-piperidin;

b) Hal bedeutet 2,6-Dichlor oder 2,6-Dibrom und R bedeutet Wasserstoff oder Methyl oder als Normaläquivalent eines Kations: 4-[3-(4-tert.Butylphenyl)-2-methylprop-1-yl]-2,6-dimethylmorpholin oder N-[3-(4-tert.Butylphenyl)-2-methylprop-1-yl]-piperidin.

Die unter die Formel I fallenden Verbindungen sind teilweise neu, teilweise bekannt. So sind in der Schweizer Patentschrift Nr 384 929 und der britischen Patentschrift Nr. 923,387 2,6-Dihaloisonicotinsäurederivate, z.B. die freie Säure und einige ihrer Ester und Salze als Herbizide beschrieben. Ferner sind in der USA-Patentschrift No. 4,137,067 und in der kanadischen Patentschrift No. 1,072,443 2,6-Dichlorisonicotinsäurealkylester als Zwischenprodukte zur Herstellung von als fungizid wirksam beschriebenen Hydrazidderivaten der vorgenannten Isonicotinsäureverbindungen angegeben. Darüber hinaus sind 2,6-Dihaloisonicotinsäure-Derivate als Tuberkulostatika bekannt geworden (vgl. Acta. Fac. Pharm. Brun. Bratislav. 4, 65-66 [1962]; Chem. Abstr. Vol 57, 1962, 4769a). Die Erfindung betrifft auch die neuen Verbindungen der Formel I, insbesondere die Salze mit organischen Basen.

Die neuen Verbindungen der vorliegenden Erfindung fallen als Untergruppen der Formel I unter folgende allgemeine Formeln:

1) Formel I'

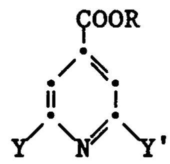

(I')

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass

1) falls Y und Y' Chlor bedeuten, R nicht Wasserstoff, $C_1$-$C_3$-Alkyl, n-Butyl, tert.Butyl, Allyl, 2-Methoxyethyl, 2-Ethoxyethyl, β-Chlorethyl, Methallyl oder durch den Rest

$COOC_4H_{9\,tert.}$

substituiertes $C_5$-Alkyl ist; oder

2) falls Y und Y' Brom bedeuten, R nicht Wasserstoff, Ethyl, β-Chlorethyl, β-Methoxyethyl oder β-Ethoxyethyl ist; oder

3) falls Y und Y' Jod bedeuten, R nicht Wasserstoff oder Ethyl ist; oder

4) falls Y und Y' Fluor bedeuten, R nicht Wasserstoff ist.

2) Formel I"

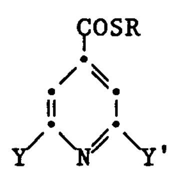

(I")

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass falls Y und Y' Chlor darstellen, R nicht Methyl oder n-Butyl ist.

3) Formel I"'

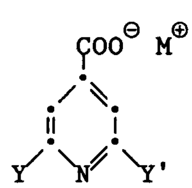

(I"')

in welcher

Y und Y' für Halogen stehen und $M^{\oplus}$ ein Normaläquivalent eines Kations darstellt mit der Massgabe, dass falls Y und Y' Chlor, Brom oder Jod bedeuten, $M^{\oplus}$ nicht $Na^{\oplus}$, $K^{\oplus}$, $1/2Ca^{\oplus}$ oder $NH_4^{\oplus}$ ist oder darüber hinaus falls Y und Y' Chlor ist, $M^{\oplus}$ nicht für $1/2Ca^{\oplus}$ steht.

4) Formel $I^{IV}$

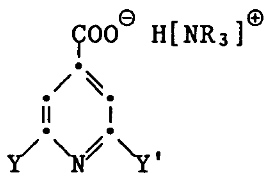

($I^{IV}$)

in welcher

Y und Y' für Halogen stehen, $[NR_3]$ ein Alkylamin mit 1 bis 3 ($C_1$-$C_6$)-Alkylgruppen oder ein durch ein oder mehrere Sauerstoffatome unterbrochenes Alkylamin mit 1 bis 3 ($C_1$-$C_6$)-Alkylgruppen darstellt oder ferner folgende cyclische Alkylamine bedeutet:

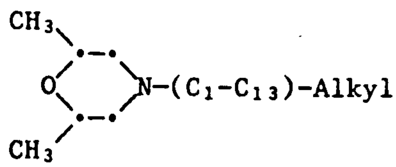 ; 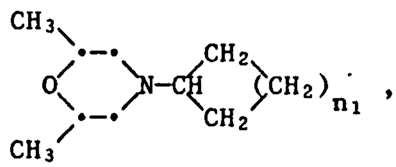 ,

worin $n_1$=1-8 ist;

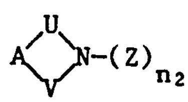 ,

worin A Sauerstoff oder die Methylgruppe, U und V $C_1$-$C_3$-Alkylen oder $C_1$-$C_3$-Alkyalkylen, Z $C_1$-$C_4$-Alkylen, Ph unsubstituiertes oder durch $C_1$-$C_4$-Alkyl substituiertes Phenyl bedeuten $n_2$ für 0 oder 1 steht, wobei die Enantiomeren der chiralen Strukturen der cyclischen Alkylamine miteingeschlossen sind.

Wegen ihrer hervorragenden biologischen Aktivität sind folgende Verbindungen bevorzugt:

<u>Gruppe A</u> (bekannte Verbindungen)

2,6-Dichlor-isonicotinsäure (Verb. 1.1);
2,6-Dichlor-isonicotinsäuremethylester (Verb. 1.2);
2,6-Dichlor-isonicotinsäureethylester (Verb. 1.3);
2,6-Dibrom-isonicotinsäure (Verb. 1.10).

<u>Gruppe B</u> (neue Verbindungen)

Salz der 2,6-Dichlor-isonicotinsäure mit N-[3-(4-tert. Butylphenyl)-2-methyl-n-prop-1-yl]-2,6-dimethylmorpholin (Verb. 4.8);
Salz der 2,6-Dichlor-isonicotinsäure mit N-[3-(4-tert.Butylphenyl)-2-methyl-n-propyl-1-yl]-piperidin (Verb. 4.9)
2,6-Dichlor-isonicotinsäurepropargylester (Verb. 1.29);
2,6-Dichlor-isonicotinsäurecyclohexylester (Verb. 1.25);
2,6-Difluor-isonicotinsäuremethylester (Verb. 1.36).

Es wurde nun überraschenderweise gefunden, dass die Verbindungen der Formel I durch ihre erfindungsgemäße Verwendung den Befall von gesunden Pflanzen durch schädliche Mikroorganismen verhindern und damit den befallsbedingten Schädigungen an Pflanzen vorbeugen. Der grosse Vorteil des erfindungsgemässen Verfahrens zur Behandlung von Pflanzen besteht darin, dass anstelle der direkten

Einwirkung von chemischen Substanzen auf die pflanzenschädigenden Mikroorganismen eine Aktivierung und Stimulierung des pflanzeneigenen biologischen Abwehrsystems vor dem Befall der Pflanzen eintritt, so dass eine Gesunderhaltung der behandelten Pflanzen aus eigener Kraft ohne weiteren direkten Einsatz mikrobizider Substanzen während der Vegetationsperiode gewährleistet werden kann. Für die Wirkstoffe der Formel I ist somit charakteristisch, dass sie keine direkte Wirkung auf die Schadorganismen ausüben, sondern stattdessen eine immunisierende Wirkung auf gesunde Pflanzen gegen Pflanzenkrankheiten besitzen. Direkte Wirkungen gegen Vertreter der wichtigsten Pilzgruppen (z.B. Fungi Imperfecti, Oomyceten, Actinomyceten) konnten nicht nachgewiesen werden. Demzufolge werden durch die erfindungsgemässe Verwendung der Verbindungen der Formel I nachteilige Nebeneffekte, wie sie sonst bei der direkten Parasitenbekämpfung auf Pflanzen durch chemische Substanzen mehr oder weniger zu beobachten sind, vermieden, was vorteilhafterweise ein völlig ungestörtes Wachstum der Pflanzen zur Folge hat.

Die der Erfindung zugrundeliegende Wirkungsweise der Verbindungen der Formel I ist gleichzeitig auf eine allgemeine Erhöhung der Abwehrbereitschaft der behandelten Pflanzen gerichtet, so dass dadurch eine generelle antimikrobielle Resistenz gegen ein breites Spektrum von schädlichen Mikroorganismen erzielt wird. Das erfindungsgemässe Verfahren ist deshalb besonders für praktische Anwendungen geeignet. Die den Verbindungen der Formel I eigene systemische Aktivität bewirkt, dass sich der Schutzeffekt auch auf zuwachsende Pflanzenteile der behandelten Pflanzen erstreckt.

Das erfindungsgemässe immunisierende Verfahren ist gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula).

Besonders vorteilhaft kann das Verfahren zur Immunisierung gegen folgende Schadorganismen eingesetzt werden:

Pilze, wie z.B. Oomyceten (z.B. Plasmopara viticola, Phytophthora infestans), Fungi imperfecti (z.B. Colletotrichum lagenarium, Piricularia oryzae, Cercospora nicotinae), Ascomyceten (z.B. Venturia inaequalis);

Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria; Erwinia (z.B. Erwinia amylovora; und

Viren, wir z.B. das Tabakmosaikvirus.

Das erfindungsgemässe Verfahren kann zum Schutz von Pflanzen aus unterschiedlichen Nutzkulturen eingesetzt werden.

Für die hierin offenbarten Indikationsgebiete gelten im Rahmen dieser Erfindung z.B. folgende Pflanzenarten:

Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen).

Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Tomate, Pfeffer, Kartoffeln, Birne und Apfel.

Die Verbindungen der Formel I können nach folgenden Verfahren über die 2,6-Dihalogen-isonicotinsäure oder deren Derivate als Zwischenprodukte hergestellt werden:

A) 2,6-Dichlor-isonicotinsäure und -derivate

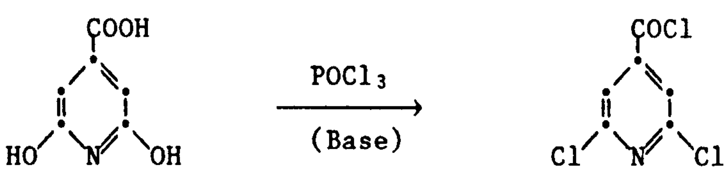

Die Umsetzung mit $POCl_3$ findet äquimolar oder im Ueberschuss unter einem Druck von $1\text{-}100 \cdot 10^5$ Pa, bevorzugt $30\text{-}100 \cdot 10^5$ Pa, und einer Temperatur von 50-160 ° C gegebenenfalls in Gegenwart einer Base statt. Als Base ist geeignet z.B. N,N-Dimethylanilin, Lutidin oder Pyridin (Lit. Houben-Weyl) 5/3, S. 925).

Anschliessend wird mit einem dem gewünschten Ester entsprechenden Alkohol verestert oder hydrolysiert.

Die Veresterung wird mit einem Alkohol im Ueberschuss bei Temperaturen von 0-80 ° C, bevorzugt 0-30 ° C, durchgeführt. Von der freien Säure ausgehend kann die Veresterung z.B. auch in Gegenwart von Dicyclohexylcarbodiimid oder mittels Carbonyldiimidazol vorgenommen werden.

Die Ester können auch direkt aus dem Reaktionsgemisch durch Solvolyse mit dem entsprechenden Alkohol erhalten werden.

B) 2,6-Dibrom-isonicotinsäure und -derivate

1.

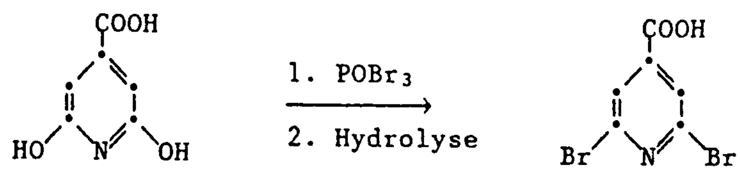


Die Reaktion findet bei Temperaturen von 50-200 ° C unter einem Druck von $1\text{-}100 \cdot 10^5$ Pa statt.

2. Durch Umhalogenierung von 2,6-Dichlorisonicotinsäurederivaten mit Alkalibromiden, z.B. NaBr oder KBr, in dipolaren aprotischen Lösungsmitteln, gegebenenfalls in Gegenwart eines Katalysators, wie z.B. eines Kronenethers (15-Krone-5-Ether, 18-Krone-6-Ether).

3. Durch Umhalogenierung von 2,6-Dichlorisonicotinsäure oder deren Derivate, wie Suaurehalogenide oder einheitliche oder gemischte Säureanhydride mit gasförmiger Bromwasserstoffsäure in einem inerten organischen Lösungsmittel, vorzugsweise einer halogenierten oder unhalogenierten Carbonsäure wie z.B. Essigsäure, Propionsäure, Trichloressigsäure, Trifluoroessigsäure oder Tribromessigsäure, bevorzugt Essigsäure, bei Temperaturen von 20 ° -150 ° C, vorzugsweise 60 ° -120 ° C, unter einem Druck von $1\text{-}100 \cdot 10^5$ Pa, vorzugsweise unter Normaldruck.

Anschliessend wird mit einem dem gewünschten Ester entsprechenden Alkohol verestert (Lit. Acta Fac. Pharm. Bohemoslovenica IV, 1962, 65) oder hydrolysiert.

C) 2,6-Dijod-isonicotinsäure und -derivate

1.

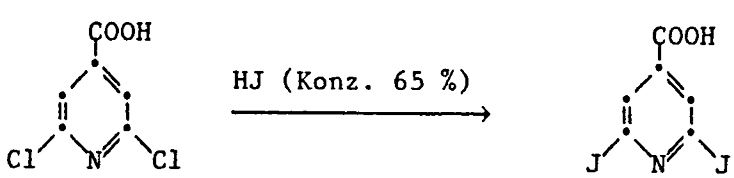


Die Reaktion wird bei einer Temperatur von über 50 ° C durchgeführt.

2. Durch Umhalogenierung von 2,6-Dichlorisonicotinsäurederivaten mit Alkalijodiden in dipolaren aprotischen Lösungsmitteln, gegebenenfalls in Gegenwart eines Katalysators, wie z.B. roten Phosphors oder eines Kronenethers.

Anschliessend wird mit einer dem gewünschten Ester entsprechenden alkoholischen Verbindung verestert (Lit. Acta. Fac. Pharm. Bohemoslovenica IV, 1962, 65) oder hydrolysiert.

D) 2,6-Difluor-isonicotinsäure und -derivate

1. Durch Umhalogenierung von 2,6-Dichlor- resp. 2,6-Dibrom-isonicotinsäurederivate mit Alkalifluoriden, vorzugsweise CsF, in dipolaren aprotischen Lösungsmitteln, wie beispielsweise Sulfolan, Dimethylsulfoxid oder Dimethylsulfon oder in einer Schmelze bei Temperaturen von 50-400 ° C.
2. Durch Erhitzen von 2,6-Dichlor-isonicotinsäurechlorid mit KF in Gegenwart von $SbF_5$ als Katalysator (gebildet aus $Sb_2O_3$ und KF) in einem Druckrohr bei Temperaturen von 100-400 ° C.
3. Durch Diazotierung von 2,6-Diaminoisonicotinsäureester in wässrigem Medium und anschliessender Umsetzung des Diazoniumsalzes in Fluorwasserstoff bei Temperaturen von 0-100 ° C, gegebenenfalls im Autoclav unter einem Druck von $1\text{-}100 \cdot 10^5$ Pa.

Anschliessend wird mit einem dem gewünschten Ester entsprechenden Alkohol verestert, wobei die Veresterung vorzugsweise ohne Isolierung des Säurefluorids direkt durchgeführt wird, oder hydrolysiert.

Als Lösungsmittel für die Umsetzungen gemäss (B2) und (C2) und (D1) kommen in Frage z.B. Dimethylformamid, Dimethylsulfoxid, Sulfolan oder Hexamethylphosphorsäuretriamid.

Die unter A-D beschriebenen Herstellungsverfahren stellen einen Teil der vorliegenden Erfindung dar. Mit der unter (B3) angegebenen Methode liegt ein neues chemisch eigenartiges Herstellungsverfahren vor.

Die Herstellung der anorganischen Salze der Formel I wird durch Umsetzung der Säuren der Formel I mit anorganischen Basen in inerten organischen Lösungsmitteln bei Temperaturen von 0 ° -150 ° C, bevorzugt 10 ° -50 ° C, durchgeführt.

Die Herstellung der organischen Salze der Formel I wird durch Umsetzung der Säuren der Formel I mit organischen Basen, wie aliphatischen oder cyclischen Aminen, in inerten organischen Lösungsmitteln bei Temperaturen von 0 ° -180 ° C, bevorzugt 10 ° -50 ° C, unter einem Druck von $1\text{-}100 \cdot 10^5$ Pa, vorzugsweise bei Normaldruck, durchgeführt.

Für den Einsatz bei den Salzbildungen kommen als inerte organische Lösungsmittel cyclische Ether, wie z.B. Dioxan oder Tetrahydrofuran, Alkohole wie z.B. Methanol, Aethanol, n-Propanol oder Isopropanol, sowie ferner dipolare aprotische Lösungsmittel, wie z.B. Dimethylformamid oder Dimethylsulfoxid, in Frage.

Die im Rahmen der Erfindung zur Anwendung gelangenden mikrobiziden Mittel, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z.B. in Form von Granulat (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen

$C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden. Besonders vorteilhafte applikationsfördernde Zuschlagstoffe sind ferner natürliche (tierische oder pflanzliche) oder synthetische Phospholipide aus der Reihe der Kephaline und Lecithine.

Als oberflächenaktive Verbindungen kommen je nach Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf.

Als nichtionische Tenside kommen in ersten Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 mit 18 Kohlenstoffatome im Alkylrest der Alkylphenols enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

RT = Raumtemperatur; DMF = Dimethylformamid; THF = Tetrahydrofuran .

1. Herstellungsbeispiele

Beispiel 1.1: Herstellung von 2,6-Dichlor-isonicotinsäureethylester

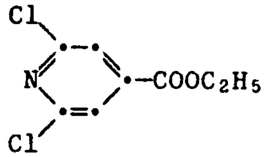

189.4 g 2,6-Dichlor-isonicotinsäurechlorid werden unter Feuchtigkeitsausschluss und Rühren bei RT zu vorgelegten 3,6 ltr. abs. Ethanol zugetropft. Dabei steigt die Innentemperatur auf 33 °C. Die Reaktion wird

unter Rühren über Nacht zu Ende geführt. Die nach dem Eindampfen verbleibende kristalline Masse (Nadeln) wird in Ether aufgenommen, mit 5%iger Natriumhydrogencarbonatlösung und Wasser gewaschen, getrocknet und eingedampft. Es wurden 191.2 g weisse Nadeln von Smp. 64-6 ° C erhalten.

Das als Ausgangsmaterial benötigte 2,6-Dichlor-isonicotinsäurechlorid wird, wie z.B. in Helv. Chim. Acta 30, 507 (1947) beschrieben, hergestellt.

Beispiel 1.2: Herstellung von 2,6-Dijod-isonicotinsäure

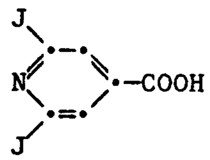

Die Verbindung wird gemäss Acta virol. 17, 326 (1971) hergestellt. Ihr Schmelzpunkt beträgt Fp. 193-195 ° C.

Beispiel 1.3: Herstellung von 2,6-Dichlor-isonicotinsäurethioethylester

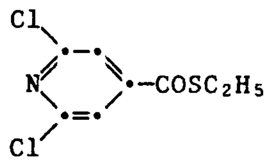

0,7 g 4-Dimethylaminopyridin und 5,0 ml Ethylmercaptan werden zu einer Lösung von 11,5 g 2,6-Dichlorisonicotinsäure in 90 ml abs. DMF gegeben. Dazu wird unter Kühlen bei 5-10 ° C eine Lösung von 12,3 g Dicyclohexylcarbodiimid in 30 ml abs. DMF zugetropft. Ueber Nacht wird bei RT gerührt, anderntags vom ausgefallenen Dicyclohexylharnstoff abfiltriert, mit DMF nachgewaschen und das Filtrat zwischen Wasser und Methylenchlorid verteilt. Die organische Phase wird mit Wasser dreimal gewaschen, über Natriumsulfat getrocknet, filtriert und eingedampft. Nach Destillation im Kugelrohr bei 150-160 ° C / 11,7 Pa resultierten 10,2 g weisse Kristalle vom Smp. 38-39 ° C.

Beispiel 1.4: Ueberführung der 2,6-Dichlor-isonicotinsäure in das Natriumsalz

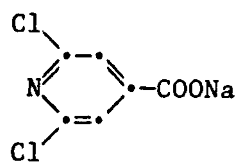

5 g 2,6-Dichlor-isonicotinsäure werden in 10 ml abs. THF gelöst und unter Kühlen mit 27,7 ml 1N Natronlauge versetzt. Nach kurzem Rühren bei RT wird eingedampft und noch vorhandene Spuren von Wasser durch mehrmalige Zugabe von Toluol und azeotrope Destillation entfernt. Das verbleibende Salz wird am Hochvakuum bei 50 ° C getrocknet. Es resultierten 5,3 g weisses Pulver; Smp. > 220 ° C.

Beispiel 1.5: Ueberführung der 2,6-Dichlor-isonicotinsäure in das Triethylammoniumsalz

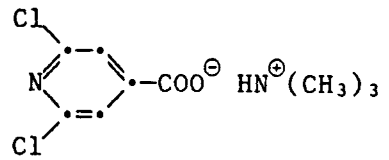

5 g 2,6-Dichlor-isonicotinsäure werden in 10 ml abs. THF gelöst. Anschliessend werden unter Kühlen tropfenweise, 3,6 ml abs. Triethylamin zugefügt und die Lösung während 10 Min., bei RT gerührt. Anschliessend wird am Rotationsverdampfer eingedampft und der verbleibende amorphe Rückstand am Hochvakuum getrocknet. Die Ausbeute des Titelproduktes betrug 6,4 g vom Smp. 101-104° C.

Beispiel 1.6: Herstellung von 2,6-Dichlor-isonicotinsäure-methylester

244 g Kaliumfluorid und 1.5 g Antimontrioxid werden gut gemischt und in möglichst dünnen Schichten abwechslungsweise mit 73.5 g 2,6-Dichlor-isonicotinsäurechlorid in ein Druckrohr eingefüllt. Der Inhalt wird 20 Std. auf 260° C erwärmt, wobei sich ein Druck von $1{,}3 \cdot 10^6$ Pa aufbaut.

Nach Abkühlung wird der Inhalt unter kräfigem Rühren und fortgesetztem Kühlen bei max. 30° C in 250 ml Methanol eingetragen und über Nacht bei RT gerührt. Dann wird filtriert, eingedampft und der Rückstand destilliert. Sdp. 81 - 82° C/$1{,}3 \cdot 10^3$ Pa.

Beispiel 1.7: Herstellung der 2,6-Difluor-isonicotinsäure

22.6 g 2,6-Difluor-isonicotinsäuremethylester werden in 25 ml Dioxan gelöst und zu einem Gemisch von 150 ml konz. Salzsäure und 100 ml Wasser gegeben. Dann wird das Gemisch 2 1/4 Stunden unter Rückfluss gekocht, das Volumen auf ca. 1/3 eingedampft und der Rückstand abgekühlt. Die kristallin anfallende Säure wird abfiltriert und getrocknet. Smp. 152 - 154° C.

Beispiel 1.8: Herstellung der 2,6-Dibromoisonicotinsäure

In die siedende Lösung von 114 g 2,6-Dichlorisonicotinsäure in 1,6 l Essigsäure*wird unter Rühren bis zur vollständigen Umsetzung gasförmige Bromwasserstoffsäure eingeleitet (im Verlauf von 2 Tagen 280 g HBr). Der Verlauf der Umsetzung wird mittels NMR-Messungen kontrolliert. Nach Abschluss der Reaktion wird das Gemisch am Vakuum eingedampft und der Rückstand mit Eiswasser versetzt. Der Niederschlag wird abfiltriert, mit Wasser gewaschen und in Dichlormethan/Tetrahydrofuran (4:1) aufgenommen. Die gebildete Lösung wird dann zweimal mit Wasser gewaschen, über Kaliumsulfat getrocknet und eingedampft. Es verbleiben 114,7 g (= 83 % der Theorie) hellbraune Kristalle von Smp. 179-187° C.

Wie in den vorstehenden Beispielen 1.1-1.8 beschrieben lassen sich folgende Verbindungen herstellen.

Verbindungen der Formel I'

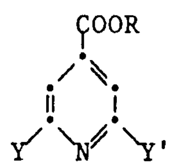

(I')

* Anstelle der reinen Essigsäure kann diese bereits vorher mit HBr auf die gewünschte Konzentration gebracht oder die handelsübliche 33%ige Lösung von HBr in Eisessig eingesetzt werden.

Tabelle la (bekannte Verbindungen)

| Verb. No. | Y | Y' | R | physik. Daten |
|---|---|---|---|---|
| 1.1 | Cl | Cl | H | Fp. 210°C |
| 1.2 | Cl | Cl | $CH_3$ | Fp. 82°C |
| 1.3 | Cl | Cl | $C_2H_5$ | Fp. 65-66°C |
| 1.4 | Cl | Cl | $C_3H_7$-n | $n_D^{20}$ 1.5257 |
| 1.5 | Cl | Cl | 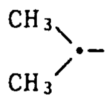 | Fp. 55-56°C |
| 1.6 | Cl | Cl | n-$C_4H_9$ | $n_D^{28}$ 1.5185 |
| 1.7 | Cl | Cl | $-CH_2CH=CH_2$ | $n_D^{28}$ 1.5200 |
| 1.8 | Cl | Cl | $-CH_2CH_2OCH_3$ | Fp. 60-61°C |
| 1.9 | Cl | Cl | $-CH_2CH_2OC_2H_5$ | Sdp. 114°C/13 Pa |
| 1.10 | Br | Br | H | Fp. 195-196°C |
| 1.11 | Br | Br | $CH_3$ | Fp. 82- 84°C |
| 1.12 | Br | Br | $C_2H_5$ | Fp. 70- 72°C |
| 1.13 | J | J | H | Fp. 193-195°C |
| 1.14 | J | J | $C_2H_5$ | Fp. 117-118°C |
| 1.15 | F | F | H | Fp. 152-154°C |

Tabelle 1b (neue Verbindungen)

| Verb. No. | Y | Y' | R | physik. Daten |
|---|---|---|---|---|
| 1.17 | Cl | Cl | s-$C_4H_9$ | |
| 1.18 | Br | Br | t-$C_4H_9$ | Fp. 34-37°C |
| 1.19 | Br | Br | n-$C_5H_{11}$ | $n_D^{20}$ 1,5508 |
| 1.20 | Br | Br | i-$C_5H_{11}$ | Fp. 62-64°C |
| 1.21 | Cl | Cl | n-$C_6H_{13}$ | |
| 1.22 | Cl | Cl | 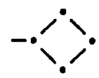 | |
| 1.23 | Cl | Cl | 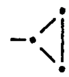 | |
| 1.24 | Cl | Cl | 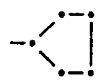 | Fp. 38-40°C |
| 1.25 | Cl | Cl | 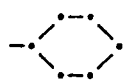 | Fp. 87-89°C |
| 1.26 | Cl | Cl | $-CH_2CH_2CH{=}CH_2$ | |
| 1.27 | Cl | Cl | 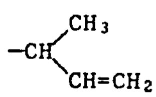 | Oel |
| 1.28 | Br | Br | $-CH_2-CH_2OC_2H_5$ | |
| 1.29 | Cl | Cl | $-CH_2C{\equiv}CH$ | Fp. 61-63°C |
| 1.30 | Cl | Cl | $-CH_2C{\equiv}CJ$ | Harz |
| 1.31 | Cl | Cl | $-CH_2CH_2C{\equiv}CH$ | |
| 1.32 | Br | Br | $C_3H_7$-n | Fp. 82-84°C |
| 1.33 | Br | Br | i-$C_3H_7$ | |
| 1.34 | Br | Br | n-$C_4H_9$ | |
| 1.35 | J | J | $CH_3$ | Fp. 122-124°C |
| 1.36 | F | F | $CH_3$ | Sdp. 81-82°C/1,3·$10^3$ Pa |
| 1.37 | F | F | $C_2H_5$ | Oel |
| 1.38 | F | F | i-$C_3H_7$ | Oel |
| 1.39 | Cl | Cl | $-CH_2CCl_3$ | Oel |
| 1.40 | Br | Br | n-$C_6H_{13}$ | |
| 1.41 | J | J | n-$C_5H_{11}$ | Oel |
| 1.42 | Br | Br | 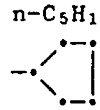 | |

Tabelle 1b (Fortsetzung)

| Verb. No. | Y | Y' | R | physik. Daten |
|---|---|---|---|---|
| 1.43 | Br | Br | 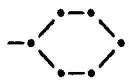 | Fp. 102-104°C |
| 1.44 | J | J | $C_3H_7$-n | Oel |
| 1.45 | Br | Br | -$CH_2$-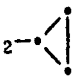 | $n_D^{20}$ 1.5752 |
| 1.46 | Cl | Cl | -$CH_2$-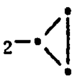 | $n_D^{20}$ 1.5415 |
| 1.47 | J | J | 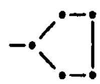 | Fp. 74-77°C |
| 1.48 | J | J | -$CH_2CH_2OCH_3$ | |
| 1.49 | Cl | Cl | -$CH(C_2H_5)_2$ | Oel |

Verbindungen der Formel I"

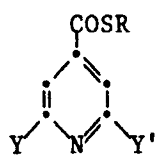

(I")

Tabelle 2a (bekannte Verbindungen)

| Verb. No. | Y | Y' | R | physik. Daten |
|---|---|---|---|---|
| 2.1 | Cl | Cl | $CH_3$ | Oel |
| 2.2 | Cl | Cl | $C_4H_9$-n | Oel |

Tabelle 2b (neue Verbindungen)

| Verb. No. | Y | Y' | R | physik. Daten |
|---|---|---|---|---|
| 2.3 | Cl | Cl | $C_2H_5$ | Fp. 38-39°C |
| 2.4 | Cl | Cl | $C_3H_7$ | $n_D^{20}$ 1.5793 |
| 2.5 | Cl | Cl | $CH_2CH=CH_2$ | |
| 2.6 | Br | Br | $CH_3$ | |
| 2.7 | Br | Br | $C_2H_5$ | |
| 2.8 | Br | Br | $C_3H_7$-n | |
| 2.9 | J | J | $CH_3$ | |
| 2.10 | J | J | $C_2H_5$ | |
| 2.11 | J | J | i-$C_3H_7$ | |
| 2.12 | F | F | $CH_3$ | Oel |
| 2.13 | F | F | $C_2H_5$ | Oel |
| 2.14 | F | F | $C_3H_7$-n | Oel |
| 2.15 | Cl | Cl | $CH_2C{\equiv}CH$ | |

Verbindungen der Formel I'''

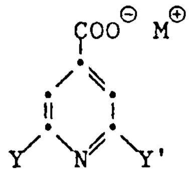

(I''')

Tabelle 3a (bekannte Verbindungen)

| Verb. No. | Y | Y' | $M^{\oplus}$ | physik. Daten |
|---|---|---|---|---|
| 3.1 | Cl | Cl | $Na^{\oplus}$ | Fp. > 220°C |
| 3.2 | Cl | Cl | 1/2 $Ca^{2\oplus}$ | Fp. > 300°C |
| 3.3 | Br | Br | $Na^{\oplus}$ | Fp. > 250°C |
| 3.4 | J | J | $Na^{\oplus}$ | Fp. > 200°C |

Tabelle 3b (neue Verbindungen)

| Verb. No. | Y | Y' | $M^{\oplus}$ | physik. Daten |
|---|---|---|---|---|
| 3.5 | Cl | Cl | $1/2\ Mg^{2\oplus}$ | Fp. > 250°C |
| 3.6 | Cl | Cl | $Li^{\oplus}$ | Fp. > 250°C |
| 3.7 | Cl | Cl | $K^{\oplus}$ | Fp. > 255-265°C (Zers.) |
| 3.8 | Br | Br | $Li^{\oplus}$ | Fp. > 200°C |
| 3.9 | Br | Br | $K^{\oplus}$ | Fp. > 250°C |
| 3.10 | Br | Br | $1/2\ Mg^{2\oplus}$ | Fp. > 250°C |
| 3.11 | J | J | $Li^{\oplus}$ | |
| 3.12 | J | J | $K^{\oplus}$ | Fp. > 220°C |
| 3.13 | F | F | $Na^{\oplus}$ | |
| 3.14 | F | F | $K^{\oplus}$ | Fp. > 210°C |
| 3.15 | F | F | $1/2\ Mg^{2\oplus}$ | |

Verbindungen der Formel $I^{IV}$

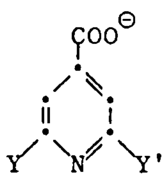 $[NR_3]H^{\oplus}$

$(I^{IV})$

Tabelle 4 (neue Verbindungen)

| Verb. No. | Y | Y' | $[NR_3]$ | physik. Daten |
|---|---|---|---|---|
| 4.1 | Cl | Cl | $N(CH_3)_3$ | |
| 4.2 | Cl | Cl | $N(C_2H_5)_3$ | Fp. 101-104°C |
| 4.3 | Cl | Cl | $N(CH_3)(C_2H_5)$ | |
| 4.4 | Cl | Cl | $N(C_2H_5)(i\text{-}C_3H_7)_2$ | |
| 4.5 | Cl | Cl | 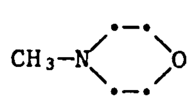 | |
| 4.6 | Cl | Cl | 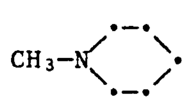 | |

Tabelle 4: (Fortsetzung)

| Verb. No. | Y | Y' | $[NR_3]H$ | physik. Daten |
|---|---|---|---|---|
| 4.7 | Cl | Cl | 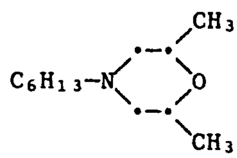 | Harz |
| 4.8 | Cl | Cl | Fm* | Fp. 155-193°C** |
| 4.9 | Cl | Cl | Fd* | Fp. 103-104°C |
| 4.10 | Cl | Cl | Tri* | |
| 4.11 | Cl | Cl | Do* | |
| 4.12 | Br | Br | $N(C_5H_5)_3$ | |
| 4.13 | Br | Br | $N(C_2H_5)(i-C_3H_7)_2$ | |
| 4.14 | Br | Br | Tri* | |
| 4.15 | Br | Br | Fd* | |
| 4.16 | Br | Br | Fm* | |
| 4.17 | J | J | $N(C_2H_5)_3$ | |
| 4.18 | J | J | Fm* | |
| 4.19 | J | J | Fd* | |
| 4.20 | F | F | $N(C_2H_5)_3$ | |
| 4.21 | F | F | Fd* | |
| 4.22 | F | F | Fm* | |
| 4.23 | Cl | Cl | $CH_3N(CH_2CH_2OC_2H_5)$ | |
| 4.24 | Cl | Cl | 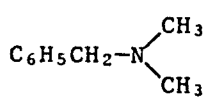 | |
| 4.25 | Cl | Cl | $C_6H_5N(CH_3)_2$ | |
| 4.26 | Cl | Cl | Cydo* | |

* Fm = 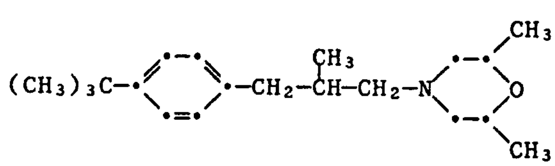

** cis-trans-Gemisch

* Fd = 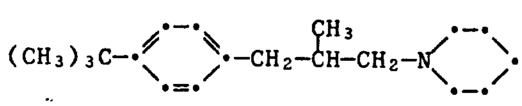

* Do = 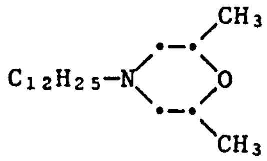

* Tri = 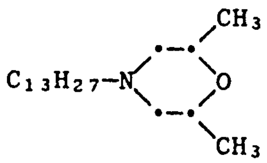

* Cydo = 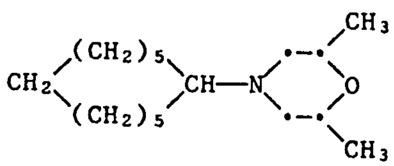

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen 1a bis 4 | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenoyl-polyethylenglykol-ether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen 1a bis 4 | 80 % | 10 % | 5 % | 95 % |
| Ethylenglykolmonomethylether | 20 % | - | - | - |
| Polyethylenglykol MG 400 | - | 70 % | - | - |

| | | | | |
|---|---|---|---|---|
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff aus den Tabellen 1a bis 4 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen 1a bis 4 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen 1a bis 4 | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |

| | | | |
|---|---|---|---|
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen 1a bis 4 | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen 1a bis 4 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.8 Extruder Granulat | |
|---|---|
| Wirkstoff aus den Tabellen 1a bis 4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.9 Umhüllungs-Granulat

| | |
|---|---|
| Wirkstoff aus den Tabellen 1a bis 4 | 3 % |
| Polyethylenglykol (MG 200) | 3 % |
| Kaolin<br>(MG = Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.10 Suspensions-Konzentrat

| | |
|---|---|
| Wirkstoff aus den Tabellen 1a bis 4 | 40 % |
| Ethylenglykol | 10 % |
| Nonylphenolpolyethylenglykolether<br>(15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen<br>wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Immunisierende Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 20 ppm).

Nach 3 Wochen werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22° bis 23 °C weitergeführt.

Die Beurteilung der Schutzwirkung erfolgt aufgrund des Pilzbefalls 7-8 Tage nach der Infektion.

Unbehandelte aber infizierte Kontrollpflanzen wiesen in dem Test einen Pilzbefall von 100 % auf.

Verbindungen aus den Tabellen 1a bis 4 bewirkten eine gute Immunisierung gegen Colletotrichum lagenarium. So blieben Pflanzen, die z.B. mit den Verbindungen Nr. 1.1, 1.2, 1.3, 1.10 oder 4.9 behandelt wurden, fast völlig frei von Colletotrichum (Befall 10 bis 0 %).

b) Gurkensamen werden mit einer Lösung des Wirkstoffes gebeizt (Konzentration: 180 g/100 kg Samen). Die Samen werden ausgesät. Nach 4 Wochen werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22° bis 23 °C weitergeführt. Die Beurteilung der Schutzwirkung erfolgt aufgrund des Pilzbefalls 7-8 Tage nach der Infektion.

Infizierte Kontrollpflanzen, deren Samen nicht behandelt wurden, wiesen in dem Test einen Pilzbefall von 100 % auf.

Verbindungen aus den Tabellen 1a bis 4 bewirkten eine gute Immunisierung gegen Colletotrichum

lagenarium. So blieben Pflanzen, deren Samen z.B. mit den Verbindungen Nr. 1.1, 1.2, 1.3, 1.10 oder 4.9 behandelt wurden, fast völlig frei von Colletotrichum (Befall 10 bis 0 %).

Beispiel 3.2: Vergleichstest (direkte Wirkung gegen Colletotrichum lagenarium)

Der formulierte Wirkstoff wird in verschiedenen Konzentrationen (10, 1, 0.1, 0.01 ppm) mit Nährboden (Kartoffeln-Karotten/Agar) gemischt. Dann werden die einzelnen Nährböden mit dem Wirkstoff in Petrischalen gegossen. Nach dem Abkühlen der Gemische wird in das Zentrum jeder Petrischale eine Myzelrondelle (Ø 8 mm) von Colletotrichum lagenarium plaziert. Die Inkubation erfolgt anschliessend bei 22°C. Nach 10 Tagen Inkubationsdauer werden die Diameter der mit Pilz bewachsenen Fläche gemessen.

Bei den Verbindungen 1.1, 1.2, 1.3, 1.4, 1.5, 1.7, 1.10, 1.11, 1.12, 1.13, 1.16, 1.29, 1.46, 2.3, 3.1, 4.2 und 4.8 wurde keine Hemmung des Pilzwachstums beobachtet. Dagegen trat bei Anwendung des Fungizids Benomyl (Handelsprodukt)

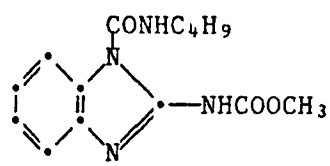

als Vergleichssubstanz bei 0,1 ppm eine 50%ige Hemmung ($EC_{50}$) von Colletotrichum lagenarium auf.

Beispiel 3.3: Immunisierende Wirkung gegen Pseudomonas lachrymans auf Cucumis sativus L.

Gurkenpflanzen werden nach 2-wöchiger Anzucht mit einer aus Spritzpulver des Wirkstoffes hergestellten Spritzbrühe besprüht (Konzentration: 20 ppm).

Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.

Die Beurteilung der Schutzwirkung erfolgt aufgrund des Bakterienbefalls 7-8 Tage nach der Infektion.

Unbehandelte aber infizierte Kontrollpflanzen wiesen in dem Test einen Krankheitsbefall von 100 % auf.

Verbindungen aus den Tabellen 1a bis 4 bewirkten eine gute Immunisierung gegen Pseudomonas lachrymans. So blieben Pflanzen, die z.B. mit den Verbindungen Nr. 1.1, 1.2, 1.3, 1.10 oder 4.9 behandelt wurden, fast völlig frei von Pseudomonas (Befall 10 bis 0 %).

Beispiel 3.4 : Vergleichstest (direkte Wirkung gegen Pseudomonas lachrymans

Der formulierte Wirkstoff wird bei verschiedenen Konzentrationen (100, 10, 1, 0.1, 0.01 ppm) mit autoklavierter und abgekühlter Nährbrühe (nutrient broth 0.8 %) gemischt und in Schalen gegossen. Anschliessend wird eine Bakteriensuspension ($10^6$ Bakterien/ml) von Pseudomonas lachrymans in die Schalen pipettiert. Die Inkubation erfolgt dann bei 22°C im Dunkeln auf einem Schütteltisch (120 rpm). Nach 10 Tagen Inkubationsdauer wird das Bakterienwachstum spektrophotometrisch bestimmt.

Bei z.B. Verbindungen 1.1 und 1.2 wurde keine Hemmung des Bakterienwachstums beobachtet. Dagegen trat bei Anwendung des Bakterizids Streptomycin als Vergleichssubstanz bei 1 ppm eine 50%ige Hemmung ($EC_{50}$) von Pseudomonas lachrymans auf.

Beispiel 3.5: Wirkung gegen Phytophthora parasitica var. nicotianae auf Tabak

Systemische Wirkung

Tabakpflanzen (8 Wochen alt) werden mit einer formulierten Lösung des Wirkungsstoffes durch Bodenapplikation behandelt (Konzentration: 2 ppm) oder injiziert (200 ppm). Nach 4 Tagen werden die Pflanzen mit Phytophthora parasitica infiziert: 2 ml einer Zoosporensuspension ($8 \cdot 10^4$ z/ml) werden um die Stengelbasis pipettiert und mit Wasser in den Boden gewaschen. Die Pflanzen werden 3 Wochen bei 24°-26°C aufbewahrt.

Die Beurteilung der Symptome erfolgt aufgrund des Welkgrades der Pflanzen.

Verbindungen aus den Tabellen 1a bis 4 zeigten gute Wirkung gegen Phytophthora parasitica. So

reduzierte z.B. die Verbindung 1.2 die Welkerscheinungen auf 0-25 %.

Unbehandelte jedoch infizierte Pflanzen waren 100%-ig verwelkt.

Beispiel 3.6: Wirkung gegen Peronospora tabacina auf Tabak

a) Residual-protektive Wirkung

Tabakpflanzen (8 Wochen alt) werden mit einer formulierten Lösung des Wirkungsstoffes besprüht (Konzentration: 200 ppm). Vier Tage nach der Behandlung werden die Pflanzen mit einer Sporangiensuspension von Peronospora tabacina ($10^4$ Sporen/ml) inokuliert, 20 Stunden im Dunkeln bei 25° C und hoher Luftfeuchtigkeit aufbewahrt und dann bei normaler Tag/Nacht Wechselfolge weiterinkubiert.

b) Systemische Wirkung

Tabakpflanzen (8 Wochen alt) werden mit einer formulierten Lösung des Wirkungsstoffes durch Bodenapplikation behandelt (Konzentration: 6 ppm). Nach 4 Tagen werden die Pflanzen mit einer Sporangiensuspension von Peronospora tabacina ($10^4$ Sporen/ml) inokuliert, 20 Stunden im Dunkeln bei 25° C und hoher Luftfeuchtigkeit aufbewahrt und dann bei normaler Tag/Nacht Wechselfolge weiterinkubiert.

Die Beurteilung der Symptome in den Tests a) und b) erfolgt aufgrund der mit Pilz befallenen Blattoberfläche.

Verbindungen aus den Tabellen 1a bis 4 zeigten gute Wirkung gegen Peronospora tabacina.

Unbehandelte jedoch infizierte Pflanze zeigten einen Befall von 90 bis 100 %.

Beispiel 3.7: Wirkung gegen Cercospora nicotianae auf Tabak

a) Residual-protektive Wirkung

Tabakpflanzen (8 Wochen alt) werden mit einer formulierten Lösung des Wirkstoffes injiziert (Konzentration: 200 ppm). Vier Tage nach der Behandlung werden die Pflanzen mit einer Sporensuspension von Cercospora nicotianae ($10^5$ Sporen/ml) inokuliert und für 5 Tage bei hoher Luftfeuchtigkeit und einer Temperatur von 22°-25° C inkubiert. Die Inkubation wird dann bei normaler Luftfeuchtigkeit und bei 20°-22° C weitergeführt.

b) Systemische Wirkung

Tabakpflanzen (8 Wochen alt) werden mit einer formulierten Lösung des Wirkungsstoffes durch Bodenapplikation behandelt (Konzentration: 20 ppm, 6 ppm, 2 ppm). Nach 4 Tagen werden die Pflanzen mit einer Sporensuspension von Cercospora nicotianae ($10^5$ Sporen/ml) inokuliert und für 5 Tage bei hoher Luftfeuchtigkeit und einer Temperatur von 22°-25° C inkubiert. Die Inkubation wird dann bei normaler Luftfeuchtigkeit und bei 20°-22° C weitergeführt.

Die Beurteilung der Symptome in den Tests (a) und (b) erfolgt aufgrund des Pilzbefalls 12 bis 14 Tage nach der Infektion.

Verbindungen aus den Tabellen 1a bis 4 zeigten gute Wirkung gegen Peronospora nicotianae. So reduzierten z.B. im Test (a) die Verbindungen 1.1, 1.2 und 1.10 den Pilzbefall auf 0-5 % und im Test (b) die Verbindungen 1.2 und 1.3 auf 0-20 %.

Die Kontrollpflanzen zeigten einen Befall von 100 %.

Beispiel 3.8: Wirkung gegen Pyricularia oryzae auf Reispflanzen

a) Residual-protektive Wirkung

Reispflanzen werden nach 2-wöchiger Anzucht mit einer aus Spritzpulver des Wirkstoffes hergestellten Spritzbrühe (0,002 % Aktivsubstanz) besprüht. Nach 72 Stunden werden die behandelten Pflanzen mit einer Konidiensuspension des Pilzes infiziert. Nach 5 Tagen Inkubation bei 95-100 % relativer Luftfeuchtigkeit und 24° C wird der Pilzbefall beurteilt.

b) Systemische Wirkung:

Zu 2-wöchigen in Töpfen verpflanzte Reispflanzen wird eine aus Spritzpulver des Wirkstoffes hergestellte Spritzbrühe gegossen (0,006 % Aktivsubstanz bezogen auf das Erdvolumen). Daraufhin werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24°C wird der Pilzbefall beurteilt.

Reispflanzen, die mit einer Spritzbrühe behandelt wurden, die als Aktivsubstanz eine Verbindung aus den Tabellen 1a bis 4 enthielt, zeigten im Vergleich zu unbehandelten Kontrollpflanzen (100 % Befall) nur geringen Pilzbefall. So reduzierte z.B. in den Tests (a) und (b) die Verbindung 1.29 den Pilzbefall auf 5 bis 20 %.

Vergleichstest (direkte Wirkung gegen Pyricularia oryzae)

Der formulierte Wirkstoff wird bei verschiedenen Konzentrationen (10, 1, 0.1 ppm) mit autoklaviertem und abgekühltem Nährboden (V-8 Gemüsesaft) gemischt und in Schalen gegossen. Anschliessend wird eine Sporensuspension (10000 Sporen/ml) in die Schalen pipettiert. Die Inkubation erfolgt dann bei 22°C im Dunkeln. Nach 2-3 Tagen wird das Pilzwachstum spektrophotometrisch bestimmt.

Bei z.B. Verbindung 1.1 wurde keine Hemmung des Wachstums von Pyricularia oryzae beobachtet.

Dagegen trat bei Anwendung des Fungizids Benomyl (Handelsprodukt; siehe Beispiel 3.2) als Vergleichssubstanz bei 0,1 ppm eine 50 %ige Hemmung ($EC_{50}$) von Pyricularia oryzae auf.

Beispiel 3.9: Wirkung gegen Pseudomonas tabaci auf Tabak

a) Residual-protektive Wirkung

Tabakpflanzen (8 Wochen alt) werden mit einer formulierten Lösung des Wirkungsstoffes injiziert (Konzentration: 200 ppm). Nach 4 Tagen werden die Pflanzen mit einer Bakteriensuspension ($2 \cdot 10^7$ Bakterien/ml) besprüht und für 3 Tage bei hoher Luftfeuchtigkeit und 22°-25°C aufbewahrt. Die Inkubation wird dann 3 Tage bei normaler Luftfeuchtigkeit und 22°-25°C weitergeführt.

b) Systemische Wirkung

Tabakpflanzen (8 Wochen alt) werden mit einer formulierten Lösung des Wirkungsstoffes durch Bodenapplikation behandelt (Konzentration: 20, 6, 2 ppm). Nach 4 Tagen werden die Pflanzen mit einer Bakteriesuspension ($2 \cdot 10^7$ Bakterien/ml) besprüht und für 3 Tage bei hoher Luftfeuchtigkeit und 22°-25°C aufbewahrt. Die Inkubation wird dann 3 Tag bei normaler Luftfeuchtigkeit und 22°-25°C weitergeführt.

Die Beurteilung der Symptome in den Tests (a) und (b) erfolgt aufgrund des Bakterienbefalls.

Unbehandelte jedoch infizierte Pflanzen zeigten einen Befall von 100 %.

Pflanzen, welche im Test (a) mit den Verbindungen 1.2 und 1.3 behandelt wurden, zeigten einen Befall von 0-20 %.

Pflanzen, welche im Test (b) mit der Verbindung 1.2 behandelt wurden, zeigten einen Befall von 0-20 %.

c) Direkte Wirkung

Der Wirkstoff wird bei verschiedenen Konzentration (100, 10, 1, 0.1 ppm) mit flüssigem Nährboden (nutrient broth), der $10^6$ Bakterien/ml enthält, gemischt und in Mikrotiterplatten gegossen. Die Platten werden bei 22°C inkubiert und das Wachstum der Bakterien wird nach 16 Stunden durch Messung der optischen Dichte bestimmt.

Bei Verwendung z.B. der Verbindungen 1.1 und 1.2 wurde keine Hemmung des Wachstums von Pseudomonas tabaci beobachtet. Dagegen verursachte Streptomycin als Vergleichssubstanz bei 0,1 ppm eine 50-prozentige Hemmung des Wachstums.

Beispiel 3.10: Wirkung gegen Tabakmosaikvirus auf Tabak

a) Immunisierende Wirkung

Tabakpflanzen (8 Wochen alt) werden mit einer formulierten Lösung des Wirkungsstoffes injiziert (Konzentration: 200 ppm). Nach 4 Tagen werden die Pflanzen mit einer Suspension von Tabakmosaikvirus

(0.5 µm/ml + Carborundum) mechanisch inokuliert und bei einer Temperatur von 20°-22°C inkubiert.

b) Direkte Wirkung

Der formulierte Wirkstoff wurde direkt zu dem Tabakmosaikvirus Inokulum addiert (200 ppm + 0.5 µg/ml Virus + Carborundum). Nach einer Stunde wurde die Mischung auf Tabakpflanzen (8 Wochen alt) mechanisch inokuliert.

Die Beurteilung der Schutzwirkung in den Tests (a) und (b) erfolgt aufgrund der Anzahl und der Grösse der Lokalläsionen 7 Tage nach der Inokulation.

Pflanzen, welche mit den Verbindungen 1.1, 1.2 behandelt wurden, zeigten im Test (a) geringfügige Läsionen (15 bis 20 %) im Vergleich mit unbehandelten jedoch infizierten Pflanzen, die einen Befall von 100 % aufwiesen.

Pflanzen, die gemäss Test (b) mit einer Mischung aus Viren und Wirkstoff inokuliert wurden, zeigten keine Schutzwirkung (100 % Befall).

**Patentansprüche**

**Patentansprüche für folgende Vertragsstaaten : BE, CH, DE, FR, GB, GR, IT, LI, LU, NL**

**1.** Verfahren zur Vorbeugung und Verhinderung des Befalls von Pflanzen durch phytopathogene Mikroorganismen, dadurch gekennzeichnet, dass auf Pflanzen und/oder deren Umgebung als Wirkstoffe Verbindungen folgender allgemeiner Formel I

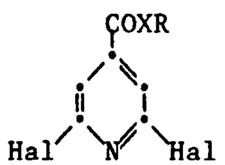

(I)

in welcher

Hal für Halogen steht,

X Sauerstoff oder Schwefel bedeutet und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl, oder ein Normaläquivalent eines Kations darstellt, das aus einer Base oder einer basischen Verbindung gebildet ist,

appliziert werden.

**2.** Verfahren gemäss Anspruch 1, dadurch gekennzeichnet, dass Hal Chlor oder Brom, X Sauerstoff und R Wasserstoff, Methyl, Ethyl, n-Propyl, iso-Propyl oder n-Butyl oder als Normaläquivalent eines Kations: Natrium, 4-[3-(4-tert.Butylphenyl)-2-methylprop-1-yl]-2,6-dimethylmorpholin oder N-[3-(4-tert.Butylphenyl)-2-methylprop1-yl]-piperidin oder 4-Cyclododecyl-2,4-dimethylmorpholin bedeuten.

**3.** Verfahren gemäss Anspruch 2, dadurch gekennzeichnet, dass Hal 2,6-Dichlor, 2,6-Dibrom oder 2,6-Dijod und R Wasserstoff oder Methyl oder als Normaläquivalent eines Kations: 4-[3-(4-tert.Butylphenyl)-2-methylprop-1-yl]-2,6-dimethylmorpholin oder n-[3-(4-tert.Butylphenyl)-2-methylprop-1-yl]-piperidin bedeuten.

**4.** Verfahren gemäss Anspruch 1, dadurch gekennzeichnet, dass Hal Chlor oder Brom, X Schwefel und R Wasserstoff, Methyl oder Ethyl oder als Normaläquivalent eines Kations: Natrium, 4-[3-(4-tert.-Butylphenyl)-2-methylprop-1-yl]-2,6-dimethylmorpholin oder N-[3-(4-tert. Butylphenyl)-2-methylprop-1-yl]-piperidin bedeuten.

**5.** Verfahren gemäss Anspruch 4, dadurch gekennzeichnet, dass Hal 2,6-Dichlor oder 2,6-Dibrom und R

Wasserstoff, Methyl oder Ethyl oder als Normaläquivalent eines Kations: 4-[3-(4-tert.Butylphenyl)-2-methylprop-1-yl ]-2,6-dimethylmorpholin oder N-[3-(4-tert.Butylphenyl)-2-methylprop-1-yl]-piperidin bedeuten.

**6.** Verfahren gemäss Anspruch 1, dadurch gekennzeichnet, dass eine Verbindung aus der Gruppe
2,6-Dichlor-isonicotinsäure (Verb. 1.1);
2,6-Dichlor-isonicotinsäuremethylester (Verb. 1.2);
2,6-Dichlor-isonicotinsäureethylester (Verb. 1.3);
2,6-Dibrom-isonicotinsäure (Verb. 1.10);
verwendet wird.

**7.** Verfahren gemäss Anspruch 1, dadurch gekennzeichnet, dass es sich bei den phytopathogenen Mikroorganismen um Pilzorganismen handelt.

**8.** Verfahren gemäss Anspruch 7, dadurch gekennzeichnet, dass es Pilzorganismen aus den Klassen Ascomycetes, Basidiomycetes oder Fungi imperfecti sind.

**9.** Verfahren gemäss Anspruch 1, dadurch gekennzeichnet, dass es sich bei den phytopathogenen Mikroorganismen um Bakterien handelt.

**10.** Verfahren gemäss Anspruch 1, dadurch gekennzeichnet, dass es sich bei den phytopathogenen Mikroorganismen um Viren handelt.

**11.** Verbindungen der Formel I'

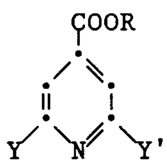

(I')

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass

1) falls Y und Y' Chlor bedeuten, R nicht Wasserstoff, $C_1$-$C_3$-Alkyl, n-Butyl, tert.Butyl, Allyl, 2-Methoxyethyl, 2-Ethoxyethyl, $\beta$-Chlorethyl, Methallyl oder durch den Rest

$$COOC_4H_{9\,tert.}$$

substituiertes $C_5$-Alkyl ist; oder

2) falls Y und Y' Brom bedeuten, R nicht Wasserstoff, Ethyl, $\beta$-Chlorethyl, $\beta$-Methoxyethyl oder $\beta$-Ethoxyethyl ist; oder

3) falls Y und Y' Jod bedeuten, R nicht Wasserstoff oder Ethyl ist; oder

4) falls Y und Y' Fluor bedeuten, R nicht Wasserstoff ist.

**12.** Verbindungen der Formel I"

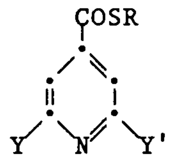

(I")

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass falls Y und Y' Chlor darstellen, R nicht Methyl oder n-Butyl ist.

**13.** Verbindungen der Formel I"'

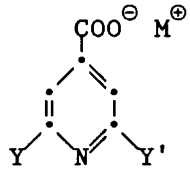

(I"')

in welcher

Y und Y' für Halogen stehen und $M^{\oplus}$ ein Normaläquivalent eines Kations darstellt mit der Massgabe, dass falls Y und Y' Chlor, Brom oder Jod bedeuten, $M^{\oplus}$ nicht $Na^{\oplus}$, $K^{\oplus}$, $1/2Ca^{\oplus}$ oder $NH_4{}^{\oplus}$ ist oder darüber hinaus falls Y und Y' Chlor ist, $M^{\oplus}$ nicht für $1/2Ca^{\oplus}$ steht.

**14.** Verbindungen der Formel $I^{IV}$

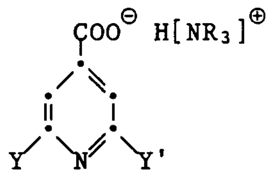

($I^{IV}$)

in welcher

Y und Y' für Halogen stehen, $[NR_3]$ ein Alkylamin mit 1 bis 3 ($C_1$-$C_6$)-Alkylgruppen oder ein durch ein oder mehrere Sauerstoffatome unterbrochenes Alkylamin mit 1 bis 3 ($C_1$-$C_6$)-Alkylgruppen darstellt oder ferner folgende cyclische Alkylamine bedeutet:

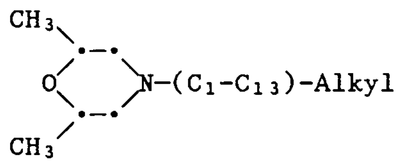 ; 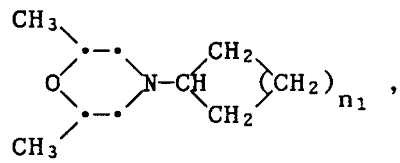 ,

worin $n_1$=1-8 ist;

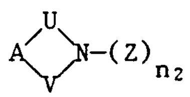 ,

worin A Sauerstoff oder die Methylengruppe, U und V $C_1$-$C_3$-Alkylen oder $C_1$-$C_3$-Alkylalkylen, Z $C_1$-$C_4$-Alkylen, Ph unsubstiuiertes oder durch $C_1$-$C_4$-Alkyl substituiertes Phenyl bedeuten und $n_2$ für 0 oder 1 steht, wobei die Enantiomeren der chiralen Strukturen der cyclischen Alkylamine miteingeschlossen sind.

**15.** Eine Verbindung aus der Gruppe:
Salz der 2,6-Dichlor-isonicotinsäure mit N-[3-(4-tert. Butylphenyl)-2-methyl-n-prop-1-yl]-2,6-dimethylmorpholin (Verb. 4.8);
Salz der 2,6-Dichlor-isonicotinsäure mit N-[3-(4-tert.Butylphenyl)-2-methyl-n-prop-1-yl]-piperidin (Verb. 4.9);
2,6-Dichlor-isonicotinsäurepropargylester (Verb. 1.29);
2,6-Dichlor-isonicotinsäurecyclohexylester (Verb. 1.25);
2,6-Difluor-isonicotinsäuremethylester (Verb. 1.36).

**16.** Verfahren zur Herstellung der Verbindung der Formel I'

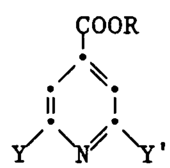 (I')

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass

1) falls Y und Y' Chlor bedeuten, R nicht Wasserstoff, $C_1$-$C_3$-Alkyl, n-Butyl, tert.Butyl, Allyl, 2-Methoxyethyl, 2-Ethoxyethyl, β-Chlorethyl, Methallyl oder durch den Rest

$COOC_4H_{9\,tert.}$

substituiertes $C_5$-Alkyl ist; oder

2) falls Y und Y' Brom bedeuten, R nicht Wasserstoff, Ethyl, β-Chlorethyl, β-Methoxyethyl oder β-Ethoxyethyl ist; oder

3) falls Y und Y' Jod bedeuten, R nicht Wasserstoff oder Ethyl ist; oder

4) falls Y und Y' Fluor bedeuten, R nicht Wasserstoff ist,

dadurch gekennzeichnet, dass man die 2,6-Dichlorisonicotinsäure, die 2,6-Dibromisonicotinsäure, die 2,6-Dijodisonicotinsäure, die Difluorisonicotinsäure oder deren Derivate in die Ester umwandelt.

**17.** Verfahren zur Herstellung der Verbindungen der Formel I"

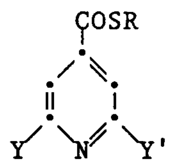

(I")

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass falls Y und Y' Chlor darstellen, R nicht Methyl oder n-Butyl ist,

dadurch gekennzeichnet, dass man die 2,6-Dichlorisonicotinsäure, die 2,6-Dibromisonicotinsäure, die 2,6-Dijodisonicotinsäure, die Difluorisonicotinsäure oder deren Derivate in die Thioester umwandelt.

**18.** Verfahren zur Herstellung der Verbindungen der Formel I"'

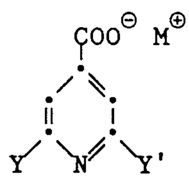

(I"')

in welcher

Y und Y' für Halogen stehen und $M^{\oplus}$ ein Normaläquivalent eines Kations darstellt mit der Massgabe, dass falls Y und Y' Chlor, Brom oder Jod bedeuten, $M^{\oplus}$ nicht $Na^{\oplus}$, $K^{\oplus}$, $1/2Ca^{\oplus}$ oder $NH_4^{\oplus}$ ist oder darüber hinaus falls Y und Y' Chlor ist, $M^{\oplus}$ nicht für $1/2Ca^{\oplus}$ steht,

dadurch gekennzeichnet, dass man die 2,6-Dichlorisonicotinsäure, die 2,6-Dibromisonicotinsäure, die 2,6-Dijodisonicotinsäure oder die Difluorisonicotinsäure in die Salze umwandelt.

**19.** Verfahren zur Herstellung der Verbindungen der Formel $I^{IV}$

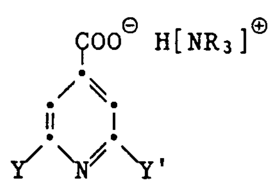

($I^{IV}$)

in welcher

Y und Y' für Halogen stehen,

$[NR_3]$ ein Alkylamin mit 1 bis 3 ($C_1$-$C_6$)-Alkylgruppen oder ein durch ein oder mehrere Sauerstoffatome unterbrochenes Alkylamin mit 1 bis 3 ($C_1$-$C_6$)-Alkylgruppen darstellt oder ferner folgende cyclische Alkylamine bedeutet:

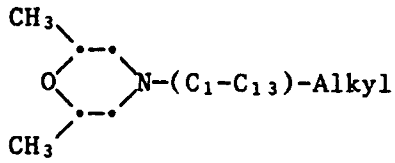 ; 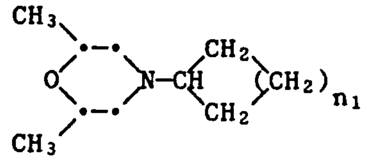 ,

worin $n_1$=1-8 ist;

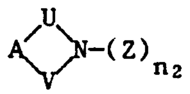 ,

worin A Sauerstoff oder die Methylengruppe, U und V $C_1$-$C_3$-Alkylen oder $C_1$-$C_3$-Alkyalkylen, Z $C_1$-$C_4$-Alkylen, Ph unsubstiuiertes oder durch $C_1$-$C_4$-Alkyl substituiertes Phenyl bedeuten und $n_2$ für 0 oder 1 steht, wobei die Enantiomeren der chiralen Strukturen der cyclischen Alkylamine miteingeschlossen sind, dadurch gekennzeichnet, dass man die 2,6-Dichlorisonicotinsäure, die 2,6-Dibromisonicotinsäure, die 2,6-Dijodisonicotinsäure oder die Difluorisonicotinsäure in die Aminsalze umwandelt.

**20.** Verfahren zur Herstellung von 2,6-Dibrom-isonicotinsäure oder deren Derivate, wie Säurehalogenide oder Säureanhydride, durch Umhalogenierung der 2,6-Dichlor-isonicotinsäure oder den entsprechenden Derivaten mit gasförmiger Bromwasserstoffsäure in einem organischen Lösungsmittel bei Temperaturen von 20°-150°C unter einem Druck von $1\text{-}100 \cdot 10^5$ Pa.

**21.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I gemäss Anspruch 1 enthält.

**22.** Mittel gemäss Anspruch 21, dadurch gekennzeichnet, dass es als Wirkstoff mindestens eine Verbindung gemäss den Ansprüchen 2-6 enthält.

**23.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I′ gemäss Anspruch 11 enthält.

**24.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I″ gemäss Anspruch 12 enthält.

**25.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I‴ gemäss Anspruch 13 enthält.

**26.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel $I^{IV}$ gemäss Anspruch 14 enthält.

**27.** Mittel gemäss Anspruch 21, dadurch gekennzeichnet, dass es als Wirkstoff eine Verbindung aus Anspruch 15 enthält.

**28.** Mittel gemäss Anspruch 21, dadurch gekennzeichnet, dass es als Wirkstoff mindestens eine Verbindung aus der Gruppe
2,6-Dichlor-isonicotinsäure (Verb. 1.1);
2,6-Dichlor-isonicotinsäuremethylester (Verb. 1.2);
2,6-Dichlor-isonicotinsäureethylester (Verb. 1.3);
2,6-Dibrom-isonicotinsäure (Verb. 1.10);
enthält.

**29.** Mittel gemäss den Ansprüchen 21-28, dadurch gekennzeichnet, dass es 0,1 bis 99 % eines Wirkstoffs

der Formel I, 99,9 bis 1 % eines festen oder flüssigen Zusatzstoffs und 0 bis 25 % eines Tensides enthält.

**30.** Mittel gemäss Anspruch 29, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

**31.** Verfahren gemäss Anspruch 1, dadurch gekennzeichnet, dass eine Verbindung aus der Gruppe:
Salz der 2,6-Dichlor-isonicotinsäure mit N-[3-(4-tert. Butylphenyl)-2-methyl-n-prop-1-yl]-2,6-dimethylmorpholin (Verb. 4.8);
Salz der 2,6-Dichlor-isonicotinsäure mit N-[3-(4-tert.Butylphenyl)-2-methyl-n-prop-1-yl]-piperidin (Verb. 4.9); 2,6-Dichlor-isonicotinsäurepropargylester (Verb. 1.29); 2,6-Dichlor-isonicotinsäurecyclohexylester (Verb. 1.25); 2,6-Difluor-isonicotinsäuremethylester (Verb. 1.36) verwendet wird.

**32.** Verwendung von Verbindungen der Formel I in Anspruch 1 zur Verbeugung und Verhinderung des Befalls von Pflanzen durch phytopathogene Mikroorganismen.

**Patentansprüche für folgende Vertragsstaaten : AT, ES**

**1.** Verfahren zur Vorbeugung und Verhinderung des Befalls von Pflanzen durch phytopathogene Mikroorganismen, dadurch gekennzeichnet, dass auf Pflanzen und/oder deren Umgebung als Wirkstoffe Verbindungen folgender allgemeiner Formel I

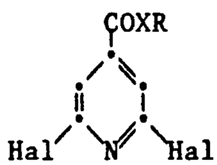

(I)

in welcher

Hal für Halogen steht,

X Sauerstoff oder Schwefel bedeutet und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl, oder ein Normaläquivalent eines Kations darstellt, das aus einer Base oder einer basischen Verbindung gebildet ist,

appliziert werden.

**2.** Verfahren gemäss Anspruch 1, dadurch gekennzeichnet, dass Hal Chlor oder Brom, X Sauerstoff und R Wasserstoff, Methyl, Ethyl, n-Propyl, iso-Propyl oder n-Butyl oder als Normaläquivalent eines Kations: Natrium, 4-[3-(4-tert.Butylphenyl)-2-methylprop-1-yl]-2,6-dimethylmorpholin oder N-[3-(4-tert.Butylphenyl)-2-methylprop-1-yl]-piperidin oder 4-Cyclododecyl-2,4-dimethylmorpholin bedeuten.

**3.** Verfahren gemäss Anspruch 2, dadurch gekennzeichnet, dass Hal 2,6-Dichlor, 2,6-Dibrom oder 2,6-Dijod und R Wasserstoff oder Methyl oder als Normaläquivalent eines Kations: 4-[3-(4-tert.Butylphenyl)-2-methylprop-1-yl]-2,6-dimethylmorpholin oder N-[3-(4-tert.Butylphenyl)-2-methylprop-1-yl]-piperidin bedeuten.

**4.** Verfahren gemäss Anspruch 1, dadurch gekennzeichnet, dass Hal Chlor oder Brom, X Schwefel und R Wasserstoff, Methyl oder Ethyl oder als Normaläquivalent eines Kations: Natrium, 4-[3-(4-tert.-Butylphenyl)-2-methylprop-1-yl]-2,6-dimethylmorpholin oder N-[3-(4-tert. Butylphenyl)-2-methylprop-1-yl]-piperidin bedeuten.

**5.** Verfahren gemäss Anspruch 4, dadurch gekennzeichnet, dass Hal 2,6-Dichlor oder 2,6-Dibrom und R Wasserstoff, Methyl oder Ethyl oder als Normaläquivalent eines Kations: 4-[3-(4-tert.Butylphenyl)-2-methylprop-1-yl ]-2,6-dimethylmorpholin oder N-[3-(4-tert.Butylphenyl)-2-methylprop-1-yl]-piperidin bedeuten.

**6.** Verfahren gemäss Anspruch 1, dadurch gekennzeichnet, dass eine Verbindung aus der Gruppe
2,6-Dichlor-isonicotinsäure (Verb. 1.1);
2,6-Dichlor-isonicotinsäuremethylester (Verb. 1.2);
2,6-Dichlor-isonicotinsäureethylester (Verb. 1.3);
2,6-Dibrom-isonicotinsäure (Verb. 1.10);
verwendet wird.

**7.** Verfahren gemäss Anspruch 1, dadurch gekennzeichnet, dass es sich bei den phytopathogenen Mikroorganismen um Pilzorganismen handelt.

**8.** Verfahren gemäss Anspruch 7, dadurch gekennzeichnet, dass es Pilzorganismen aus den Klassen Ascomycetes, Basidiomycetes oder Fungi imperfecti sind.

**9.** Verfahren gemäss Anspruch 1, dadurch gekennzeichnet, dass es sich bei den phytopathogenen Mikroorganismen um Bakterien handelt.

**10.** Verfahren gemäss Anspruch 1, dadurch gekennzeichnet, dass es sich bei den phytopathogenen Mikroorganismen um Viren handelt.

**11.** Verfahren zur Herstellung der Verbindung der Formel I'

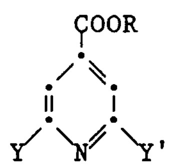

(I')

in welcher
Y und Y' für Halogen stehen und
R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass
1) falls Y und Y' Chlor bedeuten, R nicht Wasserstoff, $C_1$-$C_3$-Alkyl, n-Butyl, tert.Butyl, Allyl, 2-Methoxyethyl, 2-Ethoxyethyl, $\beta$-Chlorethyl, Methallyl oder durch den Rest

$$COOC_4H_{9\,tert.}$$

substituiertes $C_5$-Alkyl ist; oder
2) falls Y und Y' Brom bedeuten, R nicht Wasserstoff, Ethyl, $\beta$-Chlorethyl, $\beta$-Methoxyethyl oder $\beta$-Ethoxyethyl ist; oder
3) falls Y und Y' Jod bedeuten, R nicht Wasserstoff oder Ethyl ist; oder
4) falls Y und Y' Fluor bedeuten, R nicht Wasserstoff ist.

dadurch gekennzeichnet, dass man die 2,6-Dichlorisonicotinsäure, die 2,6-Dibromisonicotinsäure, die 2,6-Dijodisonicotinsäure, die Difluorisonicotinsäure oder deren Derivate in die Ester umwandelt.

**12.** Verfahren zur Herstellung der Verbindungen der Formel I"

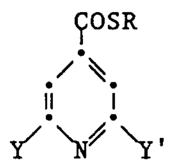

(I")

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass falls Y und Y' Chlor darstellen, R nicht Methyl oder n-Butyl ist,

dadurch gekennzeichnet, dass man die 2,6-Dichlorisonicotinsäure, die 2,6-Dibromisonicotinsäure, die 2,6-Dijodisonicotinsäure, die Difluorisonicotinsäure oder deren Derivate in die Thioester umwandelt.

**13.** Verfahren zur Herstellung der Verbindungen der Formel I"'

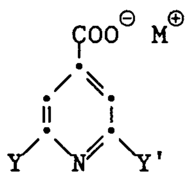

(I"')

in welcher

Y und Y' für Halogen stehen und $M^{\oplus}$ ein Normaläquivalent eines Kations darstellt mit der Massgabe, dass falls Y und Y' Chlor, Brom oder Jod bedeuten, $M^{\oplus}$ nicht $Na^{\oplus}$, $K^{\oplus}$, $1/2Ca^{\oplus}$ oder $NH_4^{\oplus}$ ist oder darüber hinaus falls Y und Y' Chlor ist, $M^{\oplus}$ nicht für $1/2Ca^{\oplus}$ steht.

dadurch gekennzeichnet, dass man die 2,6-Dichlorisonicotinsäure, die 2,6-Dibromisonicotinsäure, die 2,6-Dijodisonicotinsäure oder die Difluorisonicotinsäure in die Salze umwandelt.

**14.** Verfahren zur Herstellung der Verbindungen der Formel $I^{IV}$

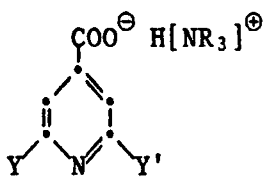

($I^{IV}$)

in welcher

Y und Y′ für Halogen stehen,

$[NR_3]$ ein Alkylamin mit 1 bis 3 ($C_1$-$C_6$)-Alkylgruppen oder ein durch ein oder mehrer Sauerstoffatome unterbrochenes Alkylamin mit 1 bis 3 ($C_1$-$C_6$)-Alkylgruppen darstellt oder ferner folgende cyclische Alkylamine bedeutet:

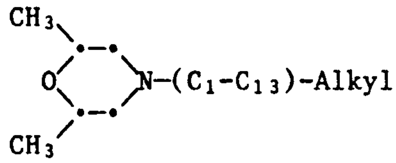 ; 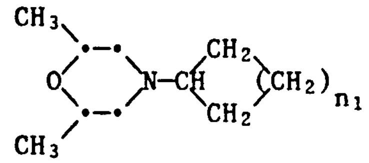 ,

worin $n_1$=1-8 ist;

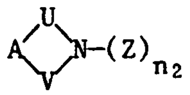 ,

worin A Sauerstoff oder die Methylengruppe, U und V $C_1$-$C_3$-Alkylen oder $C_1$-$C_3$-Alkyalkylen, Z $C_1$-$C_4$-Alkylen, Ph unsubstituiertes oder durch $C_1$-$C_4$-Alkyl substituiertes Phenyl bedeuten und $n_2$ für 0 oder 1 steht, wobei die Enantiomeren der chiralen Strukturen der cyclischen Alkylamine miteingeschlossen sind, dadurch gekennzeichnet, dass man die 2,6-Dichlorisonicotinsäure, die 2,6-Dibromisonicotinsäure, die 2,6-Dijodisonicotinsäure oder die Difluorisonicotinsäure in die Aminsalze umwandelt.

**15.** Verfahren zur Herstellung von 2,6-Dibrom-isonicotinsäure oder deren Derivate, wie Säurehalogenide oder Säureanhydride, durch Umhalogenierung der 2,6-Dichlor-isonicotinsäure oder den entsprechenden Derivaten mit gasförmiger Bromwasserstoffsäure in einem organischen Lösungsmittel bei Temperaturen von 20°-150°C unter einem Druck von 1-100•$10^5$ Pa.

**16.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I gemäss Anspruch 1 enthält.

**17.** Mittel gemäss Anspruch 16, dadurch gekennzeichnet, dass es als Wirkstoff mindestens eine Verbindung gemäss den Ansprüchen 2-6 enthält.

**18.** Mittel zur Vorbeugung und Verhinderung des Befalls von Pflanzen durch phytopathogene Mikroorganismen, dadurch gegekennzeichnet, dass es als Wirkstoff mindestens eine Verbindung der Formel I'

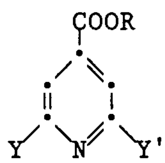 (I')

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass

1) falls Y und Y' Chlor bedeuten, R nicht Wasserstoff, $C_1$-$C_3$-Alkyl, n-Butyl, tert.Butyl, Allyl, 2-Methoxyethyl, 2-Ethoxyethyl, β-Chlorethyl, Methallyl oder durch den Rest

$COOC_4H_{9\,tert.}$

substituiertes $C_5$-Alkyl ist; oder

2) falls Y und Y' Brom bedeuten, R nicht Wasserstoff, Ethyl, β-Chlorethyl, β-Methoxyethyl oder β-Ethoxyethyl ist; oder

3) falls Y und Y' Jod bedeuten, R nicht Wasserstoff oder Ethyl ist; oder

4) falls Y und Y' Fluor bedeuten, R nicht Wasserstoff ist,

enthält.

**19.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I"

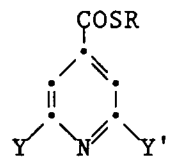

(I")

in welcher

Y und Y' für Halogen stehen und

R Wasserstoff, $C_1$-$C_6$-Alkyl, durch ein Sauerstoff- oder Schwefelatom unterbrochenes $C_1$-$C_6$-Alkyl, durch Halogen, Cyano oder den Rest COO-$C_1$-$C_6$-Alkyl substituiertes $C_1$-$C_6$-Alkyl, $C_3$-$C_5$-Alkenyl oder durch Halogen substituiertes $C_3$-$C_5$-Alkenyl, $C_3$-$C_5$-Alkinyl oder durch Halogen substituiertes $C_3$-$C_5$-Alkinyl, $C_3$-$C_6$-Cycloalkyl oder durch Halogen oder Methyl substituiertes $C_3$-$C_6$-Cycloalkyl darstellt mit der Massgabe, dass falls Y und Y' Chlor darstellen, R nicht Methyl oder n-Butyl ist,

enthält.

**20.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel I"'

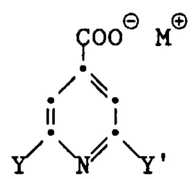

(I"')

in welcher

Y und Y' für Halogen stehen und $M^{\oplus}$ ein Normaläquivalent eines Kations darstellt mit der Massgabe, dass falls Y und Y' Chlor, Brom oder Jod bedeuten, $M^{\oplus}$ nicht $Na^{\oplus}$, $K^{\oplus}$, $1/2Ca^{\oplus}$ oder $NH_4^{\oplus}$ ist oder darüber hinaus falls Y und Y' Chlor ist, $M^{\oplus}$ nicht für $1/2Ca^{\oplus}$ steht,

enthält.

**21.** Mittel zur Vorbeugung und Verhinderung des Befalls von Pflanzen durch phytopathogene Mikroorganismen, dadurch gekennzeichnet, dass es als Wirkstoff mindestens eine Verbindung der Formel $I^{IV}$

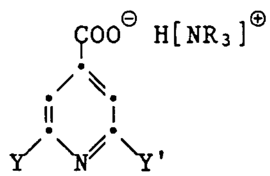

($I^{IV}$)

in welcher

Y und Y' für Halogen stehen, $[NR_3]$ ein Alkylamin mit 1 bis 3 ($C_1$-$C_6$)-Alkylgruppen oder ein durch ein

oder mehrere Sauerstoffatome unterbrochenes Alkylamin mit 1 bis 3 ($C_1$-$C_6$)-Alkylgruppen darstellt oder ferner folgende cyclische Alkylamine bedeutet:

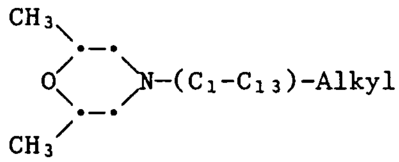 ; 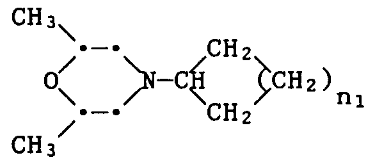 ,

worin $n_1$=1-8 ist;

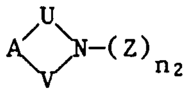 ,

worin A Sauerstoff oder die Methylengruppe, U und V $C_1$-$C_3$-Alkylen oder $C_1$-$C_3$-Alkyalkylen, Z $C_1$-$C_4$-Alkylen, Ph unsubstituiertes oder durch $C_1$-$C_4$-Alkyl substitueirtes Phenyl bedeuten und $n_2$ für 0 oder 1 steht, wobei die Enantiomeren der chiralen Strukturen der cyclischen Alkylamine miteingeschlossen sind, enthält.

**22.** Mittel zur Vorbeugung und Verhinderung des Befalls von Pflanzen durch phytopathogene Mikroorganismen, dadurch gekennzeichnet, dass es als Wirkstoff mindestens eine Verbindung aus der Gruppe:
Salz der 2,6-Dichlor-isonicotinsäure mit N-[3-(4-tert. Butylphenyl)-2-methyl-n-propyl-yl]-2,6-dimethylmorpholin (Verb. 4.8);
Salz der 2,6-Dichlor-isonicotinsäure mit N-[3-(4-tert.Butylphenyl)-2-methyl-n-prop-1-yl]-piperidin (Verb. 4.9);
2,6-Dichlor-isonicotinsäurepropargylester (Verb. 1.29);
2,6-Dichlor-isonicotinsäurecyclohexylester (Verb. 1.25);
2,6-Difluor-isonicotinsäuremethylester (Verb. 1.36),
enthält.

**23.** Mittel gemäss Anspruch 16, dadurch gekennzeichnet, dass es als Wirkstoff mindestens eine Verbindung aus der Gruppe
2,6-Dichlor-isonicotinsäure (Verb. 1.1);
2,6-Dichlor-isonicotinsäuremethylester (Verb. 1.2);
2,6-Dichlor-isonicotinsäureethylester (Verb. 1.3);
2,6-Dibrom-isonicotinsäure (Verb. 1.10);
enthält.

**24.** Mittel gemäss den Ansprüchen 16-23, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

**25.** Mittel gemäss Anspruch 24, dadurch gekennzeichnet, dass es 0,1 bis 95 % eines Wirkstoffes der Formel I, 99,8 bis 5 % eines festen oder flüssigen Zusatzstoffes und 0,1 bis 25 % eines Tensides enthält.

**26.** Verfahren gemäss Anspruch 1, dadurch gekennzeichnet, dass eine Verbindung aus der Gruppe:
Salz der 2,6-Dichlor-isonicotinsäure mit N-[3-(4-tert. Butylphenyl)-2-methyl-n-prop-1-yl]-2,6-dimethylmorpholin (Verb. 4.8);
Salz der 2,6-Dichlor-isonicotinsäure mit N-[3-(4-tert.Butylphenyl)-2-methyl-n-prop-1-yl]-piperidin (Verb. 4.9); 2,6-Dichlor-isonicotinsäurepropargylester (Verb. 1.29); 2,6-Dichlor-isonicotinsäurecyclohexylester (Verb. 1.25); 2,6-Difluor-isonicotinsäuremethylester (Verb. 1.36) verwendet wird.

**27.** Verwendung von Verbindungen der Formel I in Anspruch 1 zur Vorbeugung und Verhinderung des Befalls von Pflanzen durch phytopathogene Mikroorganismen.

## Claims

**Claims for the following Contracting States: BE, CH, DE, FR, GB, GR, IT, LI, LU, NL**

**1.** A process for protection against and prevention of infestation of plants by phytopathogenic microorganisms, which comprises applying compounds of the following general formula I

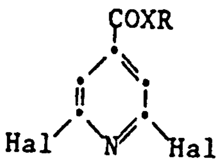

(I)

in which

Hal is halogen,

X is oxygen or sulfur, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, $C_3$-$C_6$cycloalkyl which is unsubstituted or substituted by halogen or methyl, or a normal equivalent of a cation which is formed from a base or a basic compound,

as active ingredients to plants and/or their environment.

**2.** A process according to claim 1, in which Hal is chlorine or bromine, X is oxygen, and R is hydrogen, methyl, ethyl, n-propyl, iso-propyl or n-butyl or, as a normal equivalent of a cation: sodium, 4-[3-(4-tert-butylphenyl)-2-methylprop-1-yl]-2,6-dimethylmorpholine, N-[3-(4-tert-butylphenyl)-2-methylprop-1-yl]-piperidine or 4-cyclododecyl-2,4-dimethylmorpholine.

**3.** A process according to claim 2, in which Hal is 2,6-dichloro, 2,6-dibromo or 2,6-diiodo, R is hydrogen or methyl or, as a normal equivalent of a cation: 4-[3-(4-tert-butylphenyl)-2-methylprop-1-yl]-2,6-dimethylmorpholine or N-[3-(4-tert-butylphenyl)-2-methylprop-1-yl]-piperidine.

**4.** A process according to claim 1, in which Hal is chlorine or bromine, X is sulfur and R is hydrogen, methyl or ethyl or, as a normal equivalent of a cation: sodium, 4-[3-(4-tert-butylphenyl)-2-methylprop-1-yl]-2,6-dimethylmorpholine or N-[3-(4-tert-butylphenyl)-2-methylprop-1-yl]-piperidine.

**5.** A process according to claim 4, in which Hal is 2,6-dichloro or 2,6-dibromo, and R is hydrogen, methyl or ethyl or, as a normal equivalent of a cation: 4-[3-(4-tert-butylphenyl)-2-methylprop-1-yl]-2,6-dimethylmorpholine or N-[3-(4-tert-butylphenyl)-2-methylprop-1-yl]-piperidine.

**6.** A process according to claim 1, which comprises using a compound from the group comprising
2,6-dichloroisonicotinic acid (comp. 1.1);
methyl 2,6-dichloroisonicotinate (comp. 1.2);
ethyl 2,6-dichloroisonicotinate (comp. 1.3);
and 2,6-dibromoisonicotinic acid (comp. 1.10).

**7.** A process according to claim 1, in which the phytopathogenic microorganisms are fungal organisms.

**8.** A process according to claim 7, in which the fungal organisms are from the classes Ascomycetes, Basidiomycetes or Fungi imperfecti.

**9.** A process according to claim 1, in which the phytopathogenic microorganisms are bacteria.

**10.** A process according to claim 1, in which the phytopathogenic microorganisms are viruses.

**11.** A compound of the formula I'

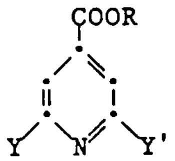

(I')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that

1) if Y and Y' are chlorine, R is not hydrogen, $C_1$-$C_3$alkyl, n-butyl, tert-butyl, allyl, 2-methoxyethyl, 2-ethoxyethyl, $\beta$-chloroethyl, methallyl, or $C_5$alkyl which is substituted by the radical COO-tert-$C_4H_9$; or

2) if Y and Y' are bromine, R is not hydrogen, ethyl, $\beta$-chloroethyl, $\beta$-methoxyethyl or $\beta$-ethoxyethyl; or

3) if Y and Y' are iodine, R is not hydrogen or ethyl; or

4) if Y and Y' are fluorine, R is not hydrogen.

**12.** A compound of the formula I"

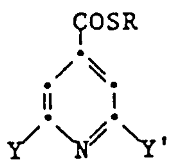

(I")

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that if Y and Y' are chlorine, R is not methyl or n-butyl.

**13.** A compound of the formula I'''

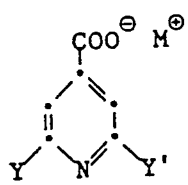

(I''')

in which

Y and Y' are halogen, and $M^{\oplus}$ is a normal equivalent of a cation, with the proviso that if Y and Y' are chlorine, bromine or iodine, $M^{\oplus}$ is not $Na^{\oplus}$, $K^{\oplus}$, $1/2Ca^{\oplus}$ or $NH_4^{\oplus}$ or, in addition, if Y and Y' are chlorine, $M^{\oplus}$ is not $1/2Ca^{\oplus}$.

**14.** A compound of the formula $I^{IV}$

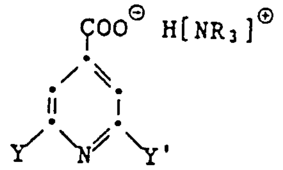

$(I^{IV})$

in which

Y and Y' are halogen, $[NR_3]$ is an alkylamine having 1 to 3 ($C_1$-$C_6$)alkyl groups or an alkylamine having 1 to 3 ($C_1$-$C_6$)alkyl groups which is interrupted by one or more oxygen atoms, or furthermore is one of the following cyclic alkylamines:

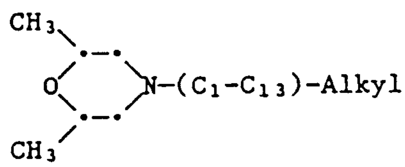 ; 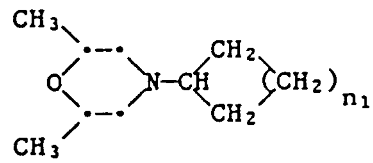 ,

in which $n_1$ is 1-8;

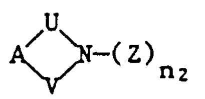 ,

in which A is oxygen or the methylene group, U and V are $C_1$-$C_3$alkylene or $C_1$-$C_3$alkylalkylene, Z is $C_1$-$C_4$alkylene, Ph is phenyl which is unsubstituted or substituted by $C_1$-$C_4$alkyl, and $n_2$ is 0 or 1, including the enantiomers of the chiral structures of the cyclic alkylamines.

**15.** A compound from the group comprising:
the salt of 2,6-dichloroisonicotinic acid with N-[3-(4-tert-butylphenyl)-2-methyl-n-prop-1-yl]-2,6-dimethylmorpholine (comp. 4.8);
the salt of 2,6-dichloroisonicotinic acid with N-[3-(4-tert-butylphenyl)-2-methyl-n-proplyl]-piperidine (comp. 4.9); propargyl 2,6-dichloroisonicotinate (comp. 1.29); cyclohexyl 2,6-dichloroisonicotinate (comp. 1.25); and methyl 2,6-difluoroisonicotinate (comp. 1.36).

**16.** A process for the preparation of a compound of the formula I'

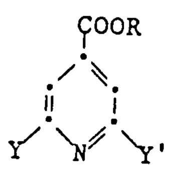

(I')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that

1) if Y and Y' are chlorine, R is not hydrogen, $C_1$-$C_3$alkyl, n-butyl, tert-butyl, allyl, 2-methoxyethyl, 2-ethoxyethyl, $\beta$-chloroethyl, methallyl or $C_5$alkyl which is substituted by the radical COO-tert-$C_4H_9$; or

2) if Y and Y' are bromine, R is not hydrogen, ethyl, β-chloroethyl, β-methoxyethyl or β-ethoxyethyl; or
3) if Y and Y' are iodine, R is not hydrogen or ethyl; or
4) if Y and Y' are fluorine, R is not hydrogen,

which comprises converting 2,6-dichloroisonicotinic acid, 2,6-dibromoisonicotinicacid, 2,6-diiodoisonicotinic acid, difluoroisonicotinic acid or a derivative thereof into the ester.

**17.** A process for the preparation of a compound of the formula I''

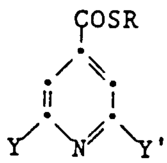

(I'')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that if Y and Y' are chlorine, R is not methyl or n-butyl,

which comprises converting 2,6-dichloroisonicotinic acid, 2,6-dibromoisonicotinicacid, 2,6-diiodoisonicotinic acid, difluoroisonicotinic acid or a derivative thereof into the thioester.

**18.** A process for the preparation of a compound of the formula I'''

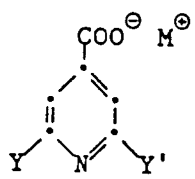

(I''')

in which

Y and Y' are halogen, and $M^{\oplus}$ is a normal equivalent of a cation, with the proviso that if Y and Y' are chlorine, bromine or iodine, $M^{\oplus}$ is not $Na^{\oplus}$, $K^{\oplus}$, $1/2Ca^{\oplus}$ or $NH_4^{\oplus}$ or, in addition, if Y and Y' are chlorine, $M^{\oplus}$ is not $1/2Ca^{\oplus}$, which comprises converting 2,6-dichloroisonicotinic acid, 2,6-dibromoisonicotinic acid, 2,6-diiodoisonicotinic acid or difluoroisonicotinic acid into the salt.

**19.** A process for the preparation of a compound of the formula $I^{IV}$

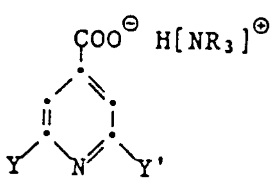

($I^{IV}$)

in which

Y and Y' are halogen, $[NR_3]$ is an alkylamine having 1 to 3 ($C_1$-$C_6$)alkyl groups or an alkylamine having

1 to 3 ($C_1$-$C_6$)alkyl groups which is interrupted by one or more oxygen atoms, or furthermore is one of the following cyclic alkylamines:

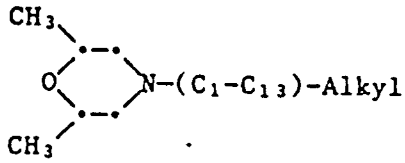 ; 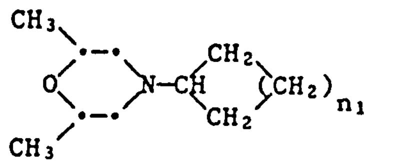 ,

in which $n_1$ is 1-8;

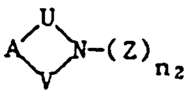 ,

in which A is oxygen or the methylene group, U and V are $C_1$-$C_3$alkylene or $C_1$-$C_3$alkylalkylene, Z is $C_1$-$C_4$alkylene, Ph is phenyl which is unsubstituted or substituted by $C_1$-$C_4$alkyl, and $n_2$ is 0 or 1, including the enantiomers of the chiral structures of the cyclic alkylamines, which comprises converting 2,6-dichloroisonicotinic acid, 2,6-dibromoisonicotinicacid, 2,6-diiodoisonicotinic acid or difluoroisonicotinic acid into the amine salt.

**20.** A process for the preparation of 2,6-dibromoisonicotinic acid or derivatives thereof, such as acid halides or acid anhydrides, by transhalogenation of 2,6-dichloroisonicotinic acid or the appropriate derivatives using gaseous hydrobromic acid in an organic solvent at temperatures of 20°-150°C under a pressure of 1-100 x $10^5$ Pa.

**21.** An agent for protection against and prevention of infestation of plants by phytopathogenic microorganisms, which contains as active ingredient at least one compound of the formula I according to claim 1.

**22.** An agent according to claim 21, which contains as active ingredient at least one compound according to any one of claims 2-6.

**23.** An agent for protection against and prevention of infestation of plants by phytopathogenic microorganisms, which contains as active ingredient at least one compound of the formula I' according to claim 11.

**24.** An agent for protection against and prevention of infestation of plants by phytopathogenic microorganisms, which contains as active ingredient at least one compound of the formula I" according to claim 12.

**25.** An agent for protection against and prevention of infestation of plants by phytopathogenic microorganisms, which contains as active ingredient at least one compound of the formula I''' according to claim 13.

**26.** An agent for protection against and prevention of infestation of plants by phytopathogenic microorganisms, which contains as active ingredient at least one compound of the formula $I^{IV}$ according to claim 14.

**27.** An agent according to claim 21, which contains as active ingredient a compound according to claim 15.

**28.** An agent according to claim 21, which contains as active ingredient at least one compound from the group comprising
2,6-dichloroisonicotinic acid (comp. 1.1);
methyl 2,6-dichloroisonicotinate (comp. 1.2);
ethyl 2,6-dichloroisonicotinate (comp. 1.3);
and 2,6-dibromoisonicotinic acid (comp. 1.10).

**29.** An agent according to any one of claims 21-28, which contains 0.1 to 99% of an active ingredient of

the formula I, 99.9 to 1% of a solid or liquid additive, and 0 to 25% of a surfactant.

**30.** An agent according to claim 29, which contains 0.1 to 95% of an active ingredient of the formula I, 99.8 to 5% of a solid or liquid additive, and 0.1 to 25% of a surfactant.

**31.** A process according to claim 1, which comprises using a compound from the group comprising:
the salt of 2,6-dichloroisonicotinic acid with N-[3-(4-tert-butylphenyl)-2-methyl-n-prop-1-yl]-2,6-dimethylmorpholine (comp. 4.8);
the salt of 2,6-dichloroisonicotinic acid with N-[3-(4-tert-butylphenyl)-2-methyl-n-prop-1-yl]-piperidine (comp. 4.9); propargyl 2,6-dichloroisonicotinate (comp. 1.29); cyclohexyl 2,6-dichloroisonicotinate (comp. 1.25); and methyl 2,6-difluoroisonicotinate (comp. 1.36).

**32.** The use of a compound of the formula I in claim 1 for protection against and prevention of infestation of plants by phytopathogenic microorganisms.

**Claims for the following Contracting States : AT, ES**

**1.** A process for protection against and prevention of infestation of plants by phytopathogenic microorganisms, which comprises applying compounds of the following general formula I

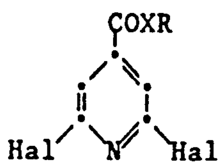

(I)

in which

Hal is halogen,

X is oxygen or sulfur, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or the COO-$C_1$-$C_6$alkyl radical, $C_3$-$C_5$alkenyl which is unsubstituted or substituted by halogen, $C_3$-$C_5$alkynyl which is unsubstituted or substituted by halogen, $C_3$-$C_6$cycloalkyl which is unsubstituted or substituted by halogen or methyl, or a normal equivalent of a cation which is formed from a base or a basic compound,

as active ingredients to plants and/or their environment.

**2.** A process according to claim 1, in which Hal is chlorine or bromine, X is oxygen, and R is hydrogen, methyl, ethyl, n-propyl, iso-propyl or n-butyl or, as a normal equivalent of a cation: sodium, 4-[3-(4-tert-butylphenyl)-2-methylprop-1-yl]-2,6-dimethylmorpholine, N-[3-(4-tert-butylphenyl)-2-methylprop-1-yl]-piperidine or 4-cyclododecyl-2,4-dimethylmorpholine.

**3.** A process according to claim 2, in which Hal is 2,6-dichloro, 2,6-dibromo or 2,6-diiodo, R is hydrogen or methyl or, as a normal equivalent of a cation: 4-[3-(4-tert-butylphenyl)-2-methylprop-1-yl]-2,6-dimethylmorpholine or N-[3-(4-tert-butylphenyl)-2-methylprop-1-yl]-piperidine.

**4.** A process according to claim 1, in which Hal is chlorine or bromine, X is sulfur and R is hydrogen, methyl or ethyl or, as a normal equivalent of a cation: sodium, 4-[3-(4-tert-butylphenyl)-2-methylprop-1-yl]-2,6-dimethylmorpholine or N-[3-(4-tert-butylphenyl)-2-methylprop-1-yl] -piperidine.

**5.** A process according to claim 4, in which Hal is 2,6-dichloro or 2,6-dibromo, and R is hydrogen, methyl or ethyl or, as a normal equivalent of a cation: 4-[3-(4-tert-butylphenyl)-2-methylprop-1-yl]-2,6-dimethylmorpholine or N-[3-(4-tert-butylphenyl)-2-methylprop-1-yl]-piperidine.

**6.** A process according to claim 1, which comprises using a compound from the group comprising

2,6-dichloroisonicotinic acid (comp. 1.1);
methyl 2,6-dichloroisonicotinate (comp. 1.2);
ethyl 2,6-dichloroisonicotinate (comp. 1.3);
and 2,6-dibromoisonicotinic acid (comp. 1.10).

**7.** A process according to claim 1, in which the phytopathogenic microorganisms are fungal organisms.

**8.** A process according to claim 7, in which the fungal organisms are from the classes Ascomycetes, Basidiomycetes or Fungi imperfecti.

**9.** A process according to claim 1, in which the phytopathogenic microorganisms are bacteria.

**10.** A process according to claim 1, in which the phytopathogenic microorganisms are viruses.

**11.** A process for the preparation of a compound of the formula I'

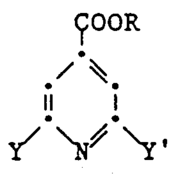

(I')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that

1) if Y and Y' are chlorine, R is not hydrogen, $C_1$-$C_3$alkyl, n-butyl, tert-butyl, allyl, 2-methoxyethyl, 2-ethoxyethyl, $\beta$-chloroethyl, methallyl or $C_5$alkyl which is substituted by the radical COO-tert-$C_4H_9$; or

2) if Y and Y' are bromine, R is not hydrogen, ethyl, $\beta$-chloroethyl, $\beta$-methoxyethyl or $\beta$-ethoxyethyl; or

3) if Y and Y' are iodine, R is not hydrogen or ethyl; or

4) if Y and Y' are fluorine, R is not hydrogen,

which comprises converting 2,6-dichloroisonicotinic acid, 2,6-dibromoisonicotinicacid, 2,6-diiodoisonicotinic acid, difluoroisonicotinic acid or a derivative thereof into the ester.

**12.** A process for the preparation of a compound of the formula I''

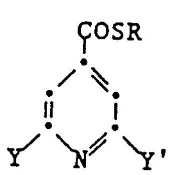

(I'')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that if Y and Y' are chlorine, R is not

methyl or n-butyl,

which comprises converting 2,6-dichloroisonicotinic acid, 2,6-dibromoisonicotinicacid, 2,6-diiodoisonicotinic acid, difluoroisonicotinic acid or a derivative thereof into the thioester.

**13.** A process for the preparation of a compound of the formula I'''

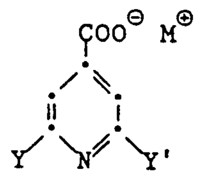

(I''')

in which

Y and Y' are halogen, and $M^{\oplus}$ is a normal equivalent of a cation, with the proviso that if Y and Y' are chlorine, bromine or iodine, $M^{\oplus}$ is not $Na^{\oplus}$, $K^{\oplus}$, $1/2Ca^{\oplus}$ or $NH_4^{\oplus}$ or, in addition, if Y and Y' are chlorine, $M^{\oplus}$ is not $1/2Ca^{\oplus}$, which comprises converting 2,6-dichloroisonicotinic acid, 2,6-dibromoisonicotinic acid, 2,6-diiodoisonicotinic acid or difluoroisonicotinic acid into the salt.

**14.** A process for the preparation of a compound of the formula $I^{IV}$

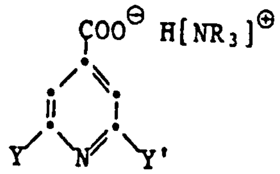

($I^{IV}$)

in which

Y and Y' are halogen, $[NR_3]$ is an alkylamine having 1 to 3 ($C_1$-$C_6$)alkyl groups or an alkylamine having 1 to 3 ($C_1$-$C_6$)alkyl groups which is interrupted by one or more oxygen atoms, or furthermore is one of the following cyclic alkylamines:

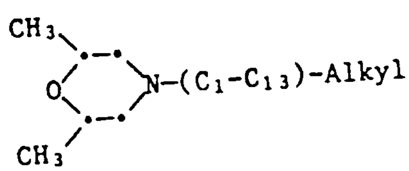 ; 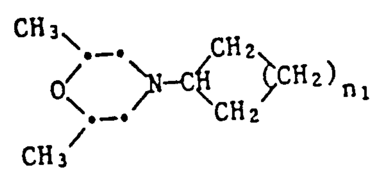 ,

in which $n_1$ is 1-8;

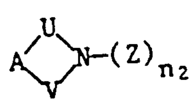 ,

in which A is oxygen or the methylene group, U and V are $C_1$-$C_3$alkylene or $C_1$-$C_3$alkylalkylene, Z is $C_1$-$C_4$alkylene, Ph is phenyl which is unsubstituted or substituted by $C_1$-$C_4$alkyl, and $n_2$ is 0 or 1, including the enantiomers of the chiral structures of the cyclic alkylamines, which comprises converting 2,6-dichloroisonicotinic acid, 2,6-dibromoisonicotinicacid, 2,6-diiodoisonicotinic acid or difluoroisonicotinic acid into the amine salt.

**15.** A process for the preparation of 2,6-dibromoisonicotinic acid or derivatives thereof, such as acid halides or acid anhydrides, by transhalogenation of 2,6-dichloroisonicotinic acid or the appropriate derivatives using gaseous hydrobromic acid in an organic solvent at temperatures of 20°-150°C under a pressure of 1-100 x $10^5$ Pa.

**16.** An agent for protection against and prevention of infestation of plants by phytopathogenic microorganisms, which contains as active ingredient at least one compound of the formula I according to claim 1.

**17.** An agent according to claim 16, which contains as active ingredient at least one compound according to any one of claims 2-6.

**18.** An agent for protection against and prevention of infestation of plants by phytopathogenic microorganisms, which contains as active ingredient at least one compound of the formula I'

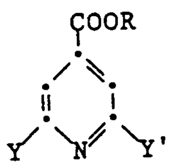

(I')

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that

1) if Y and Y' are chlorine, R is not hydrogen, $C_1$-$C_3$alkyl, n-butyl, tert-butyl, allyl, 2-methoxyethyl, 2-ethoxyethyl, β-chloroethyl, methallyl or $C_5$alkyl which is substituted by the radical COO-tert-$C_4H_9$; or

2) if Y and Y' are bromine, R is not hydrogen, ethyl, β-chloroethyl, β-methoxyethyl or β-ethoxyethyl; or

3) if Y and Y' are iodine, R is not hydrogen or ethyl; or

4) if Y and Y' are fluorine, R is not hydrogen.

**19.** An agent for protection against and prevention of infestation of plants by phytopathogenic microorganisms, which contains as active ingredient at least one compound of the formula I"

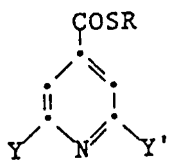

(I")

in which

Y and Y' are halogen, and

R is hydrogen, $C_1$-$C_6$alkyl, $C_1$-$C_6$alkyl which is interrupted by an oxygen or sulfur atom, $C_1$-$C_6$alkyl which is substituted by halogen, cyano or a COO-$C_1$-$C_6$alkyl radical, $C_3$-$C_5$alkenyl which is unsubstituted or substituted by halogen, $C_3$-$C_5$alkynyl which is unsubstituted or substituted by halogen, or $C_3$-$C_6$cycloalkyl which is unsubstituted or substituted by halogen or methyl, with the proviso that if Y and Y' are chlorine, R is not methyl or n-butyl.

**20.** An agent for protection against and prevention of infestation of plants by phytopathogenic microorganisms, which contains as active ingredient at least one compound of the formula I'''

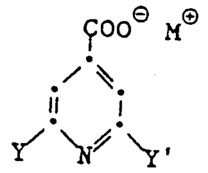

(I''')

in which

Y and Y' are halogen, and $M^{\oplus}$ is a normal equivalent of a cation, with the proviso that if Y and Y' are chlorine, bromine or iodine, $M^{\oplus}$ is not $Na^{\oplus}$, $K^{\oplus}$, $1/2Ca^{\oplus}$ or $NH_4^{\oplus}$ or, in addition, if Y and Y' are chlorine, $M^{\oplus}$ is not $1/2Ca^{\oplus}$.

**21.** An agent for protection against and prevention of infestation of plants by phytopathogenic microorganisms, which contains as active ingredient at least one compound of the formula $I^{IV}$

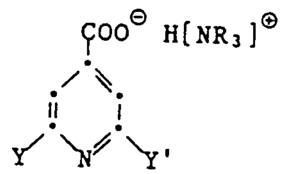

($I^{IV}$)

in which

Y and Y' are halogen, $[NR_3]$ is an alkylamine having 1 to 3 $(C_1$-$C_6)$alkyl groups or an alkylamine having 1 to 3 $(C_1$-$C_6)$alkyl groups which is interrupted by one or more oxygen atoms, or furthermore is one of the following cyclic alkylamines:

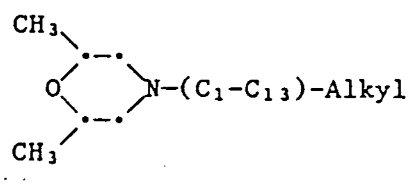

;

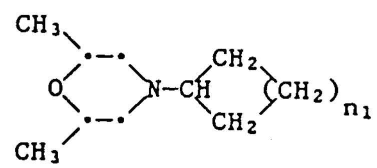

,

in which $n_1$ is 1-8;

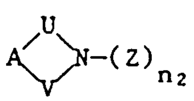

,

in which A is oxygen or the methylene group, U and V are $C_1$-$C_3$alkylene or $C_1$-$C_3$alkylalkylene, Z is $C_1$-$C_4$alkylene, Ph is phenyl which is unsubstituted or substituted by $C_1$-$C_4$alkyl, and $n_2$ is 0 or 1, including the enantiomers of the chiral structures of the cyclic alkylamines.

**22.** An agent for protection against and prevention of infestation of plants by phytopathogenic microorganisms, which contains as active ingredient at least one compound from the group comprising:

the salt of 2,6-dichloroisonicotinic acid with N-[3-(4-tert-butylphenyl)-2-methyl-n-prop-1-yl]-2,6-dimethylmorpholine (comp. 4.8);

the salt of 2,6-dichloroisonicotinic acid with N-[3-(4-tert-butylphenyl)-2-methyl-n-prop-1-yl]-piperidine (comp. 4.9);

propargyl 2,6-dichloroisonicotinate (comp. 1.29);

cyclohexyl 2,6-dichloroisonicotinate (comp. 1.25);

and methyl 2,6-difluoroisonicotinate (comp. 1.36).

**23.** An agent according to claim 16, which contains as active ingredient at least one compound from the

group comprising
2,6-dichloroisonicotinic acid (comp. 1.1);
methyl 2,6-dichloroisonicotinate (comp. 1.2);
ethyl 2,6-dichloroisonicotinate (comp. 1.3);
and 2,6-dibromoisonicotinic acid (comp. 1.10).

**24.** An agent according to any one of claims 16-23, which contains 0.1 to 99% of an active ingredient of the formula I, 99.9 to 1% of a solid or liquid additive, and 0 to 25% of a surfactant.

**25.** An agent according to claim 24, which contains 0.1 to 95% of an active ingredient of the formula I, 99.8 to 5% of a solid or liquid additive, and 0.1 to 25% of a surfactant.

**26.** A process according to claim 1, which comprises using a compound from the group comprising:
the salt of 2,6-dichloroisonicotinic acid with N-[3-(4-tert-butylphenyl)-2-methyl-n-prop-1-yl]-2,6-dimethylmorpholine (comp. 4.8);
the salt of 2,6-dichloroisonicotinic acid with N-[3-(4-tert-butylphenyl)-2-methyl-n-prop-1-yl]-piperidine (comp. 4.9); propargyl 2,6-dichloroisonicotinate (comp. 1.29); cyclohexyl 2,6-dichloroisonicotinate (comp. 1.25); and methyl 2,6-difluoroisonicotinate (comp. 1.36).

**27.** The use of a compound of the formula I in claim 1 for protection against and prevention of infestation of plants by phytopathogenic microorganisms.

**Revendications**
**Revendications pour les Etats contractants suivants : BE, CH, DE, FR, GB, GR, IT, LI, LU, NL**

**1.** Procédé pour prévenir et empêcher l'infestation des plantes par des microorganismes phytopathogènes, caractérisé en ce que l'on applique sur les plantes et/ou leur environnement, comme substances actives, les composés de formule générale I suivantes :

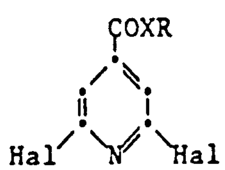

(I)

dans laquelle

Hal représente un halogène,

X représente l'oxygène ou le soufre et

R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou le méthyle ou un équivalent normal d'un cation, formé à partir d'une base ou d'un composé basique.

**2.** Procédé selon la revendication 1, caractérisé en ce que Hal représente le chlore ou le brome, X représente l'oxygène et R l'hydrogène, le méthyle, l'éthyle, le n-propyle, l'iso-propyle, ou le n-butyle ou comme équivalent normal d'un cation : le sodium, la 4-[3-(4-tert-butylphényl)-2-méthylprop-1-yl]-2,6-diméthylmorpholine ou la N-[3-(4-tert-butylphényl)-2-méthylprop-1-yl]-pipéridine ou la 4-cyclododécyl-2,4-diméthylmorpholine.

**3.** Procédé selon la revendication 2, caractérisé en ce que Hal représente le 2,6-dichloro, le 2,6-dibromo ou le 2,6-diiodo et R représente l'hydrogène ou le méthyle ou comme équivalent normal d'un cation : la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**4.** Procédé selon la revendication 1, caractérisé en ce que Hal représente le chlore ou le brome, X le soufre et R l'hydrogène, le méthyle ou l'éthyle ou comme équivalent normal d'un cation : le sodium, la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**5.** Procédé selon la revendication 4, caractérisé en ce que Hal représente le 2,6-dichloro ou le 2,6-dibromo et R représente l'hydrogène, le méthyle ou l'éthyle ou comme équivalent normal d'un cation : la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**6.** Procédé selon la revendication 1, caractérisé en ce que l'on utilise un composé du groupe
acide 2,6-dichloro-isonicotinique (comp. 1.1);
ester méthylique de l'acide 2,6-dichloro-isonicotinique (comp. 1.2);
ester éthylique de l'acide 2,6-dichloro-isonicotinique (comp. 1.3);
acide 2,6-dibromo-isonicotinique (comp. 1.10).

**7.** Procédé selon la revendication 1, caractérisé en ce que les microorganismes phytopathogènes sont des organismes provenant du champignon.

**8.** Procédé selon la revendication 7, caractérisé en ce qu'ils sont des organismes provenant des champignons des classes des ascomycètes, basidiomycètes ou Fungi imperfecti.

**9.** Procédé selon la revendication 1, caractérisé en ce que les microorganismes phytopathogènes sont des bactéries.

**10.** Procédé selon la revendication 1, caractérisé en ce que les microorganismes phytopathogènes sont des virus.

**11.** Composés de formule I'

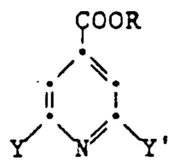

(I')

dans laquelle

Y et Y' représentent un halogène et

R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, par un cyano ou par le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou par un alcynyle en $C_3$-$C_5$ substitué par un halogène, un cycloalkyle en $C_3$-$C_6$ ou un cycloalkyle en $C_3$-$C_6$ substitué par un halogène ou le méthyle, sous réserve que

1) lorsque Y et Y' représentent le chlore, R n'est pas l'hydrogène, un alkyle en $C_1$-$C_3$, le n-butyle, le tert-butyle, l'allyle, le 2-méthoxyéthyle, le 2-éthoxyéthyle, le $\beta$-chloroéthyle, méthallyle ou un alkyle en $C_5$ substitué par le reste $COOC_4H_9$ tert.; ou

2) lorsque Y et Y' représentent le brome, R n'est pas l'hydrogène, l'éthyle, le $\beta$-chloroéthyle, le $\beta$-méthoxyéthyle ou le $\beta$-éthoxyéthyle; ou

3) lorsque Y et Y' représentent l'iode, R n'est pas l'hydrogène ou l'éthyle; ou

4) lorsque Y et Y' représentent le fluor, R n'est pas l'hydrogène.

**12.** Composés de formule I"

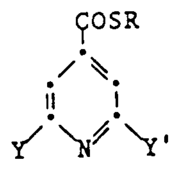

(I")

dans laquelle

Y et Y' représentent un halogène et

R représente l'hydrogène, un alkyle en $C_1$-$C_6$,un alkyle en $C_1$-$C_6$ interrompu par un atome d'oxygène ou par un atome de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou par le méthyle, sous réserve que, lorsque Y et Y' représentent le chlore, R n'est pas le méthyle ou le n-butyle.

**13.** Composés de formule I'''

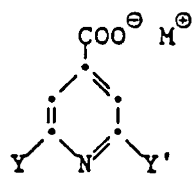

(I''')

dans laquelle

Y et Y' représentent un halogène et $M^{\oplus}$ représente un équivalent normal d'un cation, sous réserve que lorsque Y et Y' représentent le chlore, le brome ou l'iode, $M^{\oplus}$ n'est pas $Na^{\oplus}$, $K^{\oplus}$, 1/2 $Ca^{\oplus}$ ou $NH_4^{\oplus}$ ou, en outre, lorsque Y et Y' sont le chlore, $M^{\oplus}$ ne représente pas 1/2 $Ca^{\oplus}$.

**14.** Composés de formule $I^{IV}$

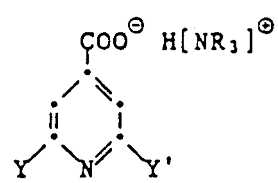

($I^{IV}$)

dans laquelle

Y et Y' représentent un halogène, $[NR_3]$ une alkylamine ayant 1 à 3 groupes alkyles en $C_1$-$C_6$ ou une alkylamine interrompue par un ou plusieurs atomes d'oxygène ayant 1 à 3 groupes alkyles en $C_1$-$C_6$ ou également les alkylamines cycliques suivantes :

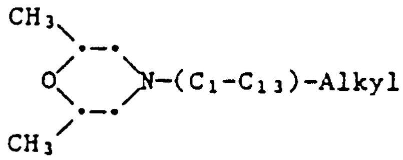 ; 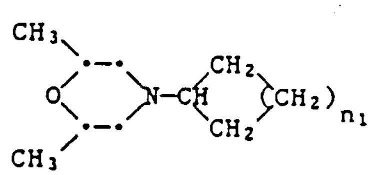

où $n_1$ = 1-8

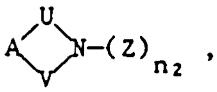

où A représente l'oxygène ou le groupe méthylène, U et V un alkylène en $C_1$-$C_3$ ou un alkyle en $C_1$-$C_3$ alkylène, Z un alkylène en $C_1$-$C_4$, Ph un phényle non substitué ou substitué par un alkyle en $C_1$-$C_4$ et $n_2$ est égal à 0 où 1, les énantiomères des structures chirales des alkylamines cycliques étant englobés.

**15.** Un composé du groupe
sel de l'acide 2,6-dichloro-isonicotinique avec la N-[3-(4-tert-butylphényl)-2-méthyl-n-prop-1-yl]-2,6-diméthylmorpholine (comp. 4.8);
sel de l'acide 2,6-dichloro-isonicotinique avec la N-[3-(4-tert-butylphényl)-2-méthyl-n-prop-1-yl]-pipéridine (comp. 4.9);
ester propargylique de l'acide 2,6-dichloro-isonicotinique (comp. 1.29);
ester cyclohexylique de l'acide 2,6-dichloro-isonicotinique (comp. 1.25);
ester méthylique de l'acide 2,6-difluoro-isonicotinique (comp. 1.36).

**16.** Procédé pour la préparation du composé de formule I'

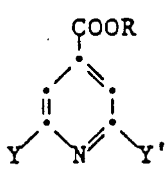

(I')

dans laquelle

Y et Y' représente un halogène et

R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, par un cyano ou par le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou par un alcynyle en $C_3$-$C_5$ substitué par un halogène, un cycloalkyle en $C_3$-$C_6$ ou un cycloalkyle en $C_3$-$C_6$ substitué par un halogène ou le méthyle, sous réserve que

1) lorsque Y et Y' représentent le chlore, R n'est pas l'hydrogène, un alkyle en $C_1$-$C_3$, le n-butyle, le tert-butyle, l'allyle, le 2-méthoxyéthyle, le 2-éthoxyéthyle, le $\beta$-chloroéthyle, le méthallyle ou un alkyle en $C_5$ substitué par le reste $COOC_4H_9$ tert.; ou
2) lorsque Y et Y' représentent le brome, R n'est pas l'hydrogène, l'éthyle, le $\beta$-chloroéthyle, le $\beta$-méthoxyéthyle ou le $\beta$-éthoxyéthyle; ou
3) lorsque Y et Y' représentent l'iode, R n'est pas l'hydrogène ou l'éthyle; ou
4) lorsque Y et Y' représentent le fluor, R n'est pas l'hydrogène,

caractérisé en ce que l'on transforme l'acide 2,6-dichloroisonicotinique, l'acide 2,6-dibromoisonicotinique, l'acide 2,6-diiodoisonicotinique, l'acide difluoroisonicotinique ou leurs dérivés en esters.

**17.** Procédé pour la préparation des composés de formule I"

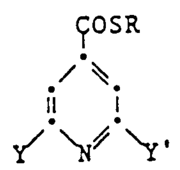

(I")

dans laquelle

Y et Y' représentent un halogène et

R représente l'hydrogène, un alkyle en $C_1$-$C_6$; un alkyle en $C_1$-$C_6$ interrompu par un atome d'oxygène ou par un atome de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou par le méthyle, sous réserve que, lorsque Y et Y' représentent le chlore, R n'est pas le méthyle ou le n-butyle,

caractérisé en ce que l'on transforme l'acide 2,6-dichloroisonicotinique, l'acide 2,6-dibromoisonicotinique, l'acide 2,6-diiodoisonicotinique, l'acide difluoroisonicotinique ou leurs dérivés en thioesters.

**18.** Procédé pour la préparation des composés de formule I'''

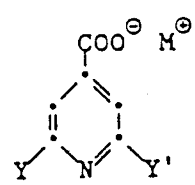

(I''')

dans laquelle

Y et Y' représente un halogène et $M^{\oplus}$ représente un équivalent normal d'un cation, sous réserve que lorsque Y et Y' représentent le chlore, le brome ou l'iode, $M^{\oplus}$ n'est pas $Na^{\oplus}$, $K^{\oplus}$, 1/2 $Ca^{\oplus}$ ou $NH_4^{\oplus}$ ou, en outre, lorsque Y et Y' sont le chlore, $M^{\oplus}$ ne représente pas 1/2 $Ca^{\oplus}$,

caractérisé en ce que l'on transforme l'acide 2,6-dichloroisonicotinique, l'acide 2,6-dibromoisonicotinique, l'acide 2,6-diiodoisonicotinique ou l'acide difluoroisonicotinique en sels.

**19.** Procédé pour la préparation des composés de formule $I^{IV}$

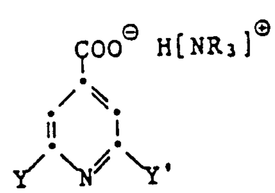

($I^{IV}$)

dans laquelle

Y et Y' représentent un halogène, $[NR_3]$ un alkylamine ayant 1 à 3 groupes alkyles en $C_1$-$C_6$ ou une alkylamine interrompue par un ou plusieurs atomes d'oxygène ayant 1 à 3 groupes alkyles en $C_1$-$C_6$ ou également les alkylamines cycliques suivantes :

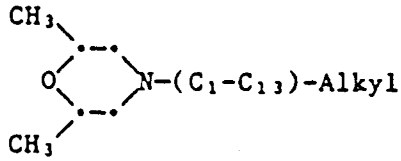 ; 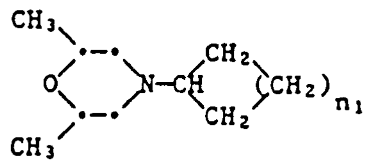 ,

où $n_1$ = 1-8

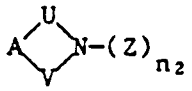 ,

où A représente l'oxygène ou le groupe méthylène, U et V un alkylène en $C_1$-$C_3$ ou un alkyle en $C_1$-$C_3$ alkylène, Z un alkylène en $C_1$-$C_4$, Ph un phényle non substitué ou substitué par un alkyle en $C_1$-$C_4$ et $n_2$ est égal à 0 ou 1, les énantiomères des structures chirales des alkylamines cycliques étant englobés,
caractérisé en ce que l'on transforme l'acide 2,6-dichloroisonicotinique, l'acide 2,6-dibromoisonicotinique, l'acide 2,6-diiodoisonicotinique ou l'acide difluoroisonicotinique en sels d'amines.

**20.** Procédé pour la préparation de l'acide 2,6-dibromo-isonicotinique ou de ses dérivés tels que les halogénures d'acide ou les anhydrides d'acide par trans-halogénation de l'acide 2,6-dichloroisonicotinique ou des dérivés correspondants avec l'acide bromhydrique gazeux dans un solvant organique à des températures de 20-150 ° C sous une pression de $1\text{-}100 \times 10^5$ Pa.

**21.** Agent pour prévenir et empêcher l'infestation des plantes par les microorganismes phytopathogènes, caractérisé en ce qu'il contient, à titre de substance active, au moins un composé de formule I selon la revendication 1.

**22.** Agent selon la revendication 21, caractérisé en ce qu'il contient, à titre de substance active, au moins un composé selon les revendications 2 à 6.

**23.** Agent pour prévenir et empêcher l'infestation des plantes par les microorganismes phytopathogènes, caractérisé en ce qu'il contient, à titre de substance active, au moins un composé de formule I' selon la revendication 11.

**24.** Agent pour prévenir et empêcher l'infestation des plantes par les microorganismes phytopathogènes, caractérisé en ce qu'il contient, à titre de substance active, au moins un composé de formule I" selon la revendication 12.

**25.** Agent pour prévenir et empêcher l'infestation des plantes par les microorganismes phytopathogènes, caractérisé, en ce qu'il contient, à titre de substance active, au moins un composé de formule I"' selon la revendication 13.

**26.** Agent pour prévenir et empêcher l'infestation des plantes par les microorganismes phytopathogènes, caractérisé en ce qu'il contient, à titre de substance active, au moins un composé de formule $I^{IV}$ selon la revendication 14.

**27.** Agent selon la revendication 21, caractérisé en ce qu'il contient, à titre de substance active, un composé de la revendication 15.

**28.** Agent selon la revendication 21, caractérisé en ce qu'il contient, à titre de substance active, au moins un composé du groupe
acide 2,6-dichloro-isonicotinique (comp. 1.1);
ester méthylique de l'acide 2,6-dichloro-isonicotinique (comp. 1.2);
ester éthylique de l'acide 2,6-dichloro-isonicotinique (comp. 1.3);
l'acide 2,6-dibromo-isonicotinique (comp. 1.10).

**29.** Agent selon les revendications 21 à 28, caractérisé en ce qu'il contient 0,1 à 99 % d'une substance

active de formule I, 99,9 à 1 % d'un additif solide ou liquide et 0 à 25 % d'un agent de surface.

**30.** Agent selon la revendication 29, caractérisé en ce qu'il contient 0,1 à 95 % d'une substance active de formule I, 99,8 % à 5 % d'un additif solide ou liquide et 0,1 à 25 % d'un agent de surface.

**31.** Procédé selon la revendication 1, caractérisé en ce que l'on utilise un composé du groupe : sel de l'acide 2,6-dichloro-isonicotinique avec la N-[3-(4-tert-butylphényl)-2-méthyl-n-prop-1-yl]-2,6-diméthylmorpholine (comp. 4.8); sel de l'acide 2,6-dichloro-isonicotinique avec la N-[3-(4-tert-butylphényl)-2-méthyl-n-prop-1-yl]-pipéridine (comp. 4.9); ester propargylique de l'acide 2,6-dichloro-isonicotinique (comp. 1.29); ester cyclohexylique de l'acide 2,6-dichloro-isonicotinique (comp. 1.25); ester méthylique de l'acide 2,6-difluoro-isonicotinique (comp. 1.36).

**32.** Utilisation des composés de formule I selon la revendication 1 pour prévenir et empêcher l'infestation des plantes par des microorganismes phytopathogènes.

**Revendications pour les Etats contractants suivants : AT, ES**

**1.** Procédé pour prévenir et empêcher l'infestation des plantes par des microorganismes phytopathogènes, caractérisé en ce que l'on applique sur les plantes et/ou leur environnement, comme substances actives, les composés de formule générale I suivantes :

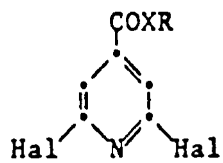

(I)

dans laquelle

Hal représente un halogène,

X représente l'oxygène ou le soufre et

R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou le méthyle ou un équivalent normal d'un cation, formé à partir d'une base ou d'un composé basique.

**2.** Procédé selon la revendication 1, caractérisé en ce que Hal représente le chlore ou le brome, X représente l'oxygène et R l'hydrogène, le méthyle, l'éthyle, le n-propyle, l'iso-propyle, ou le n-butyle ou comme équivalent normal d'un cation : le sodium, la 4-[3-(4-tert-butylphényl)-2-méthylprop-1-yl]-2,6-diméthylmorpholine ou la N-[3-(4-tert-butylphényl)-2-méthylprop-1-yl]-pipéridine ou la 4-cyclododécyl-2,4-diméthylmorpholine.

**3.** Procédé selon la revendication 2, caractérisé en ce que Hal représente le 2,6-dichloro, le 2,6-dibromo ou le 2,6-diiodo et R représente l'hydrogène ou le méthyle ou comme équivalent normal d'un cation : la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**4.** Procédé selon la revendication 1, caractérisé en ce que Hal représente le chlore ou le brome, X le soufre et R l'hydrogène, le méthyle ou l'éthyle ou comme équivalent normal d'un cation : le sodium, la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**5.** Procédé selon la revendication 4, caractérisé en ce que Hal représente le 2,6-dichloro ou le 2,6-dibromo et R représente l'hydrogène, le méthyle ou l'éthyle ou comme équivalent normal d'un cation :

la 4-[3-(4-tert-butylphényl)-2-méthylprop-1-yl]-2,6-diméthylmorpholine ou la N-[3-(4-tert-butylphényl)-2-méthylprop-1-yl]-pipéridine.

**6.** Procédé selon la revendication 1, caractérisé en ce que l'on utilise un composé du groupe
acide 2,6-dichloro-isonicotinique (comp. 1.1);
ester méthylique de l'acide 2,6-dichloro-isonicotinique (comp. 1.2);
ester éthylique de l'acide 2,6-dichloro-isonicotinique (comp. 1.3);
acide 2,6-dibromo-isonicotinique (comp. 1.10).

**7.** Procédé selon la revendication 1, caractérisé en ce que les microorganismes phytopathogènes sont des organismes provenant du champignon.

**8.** Procédé selon la revendication 7, caractérisé en ce qu'ils sont des organismes provenant des champignons des classes des ascomycètes, basidiomycètes ou Fungi imperfecti.

**9.** Procédé selon la revendication 1, caractérisé en ce que les microorganismes phytopathogènes sont des bactéries.

**10.** Procédé selon la revendication 1, caractérisé en ce que les microorganismes phytopathogènes sont des virus.

**11.** Procédé pour la préparation du composé de formule I'

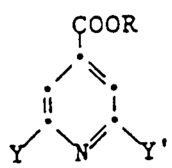

(I')

dans laquelle
Y et Y' représentent un halogène et
R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, par un cyano ou par le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou par un alcynyle en $C_3$-$C_5$ substitué par un halogène, un cycloalkyle en $C_3$-$C_6$ ou un cycloalkyle en $C_3$-$C_6$ substitué par un halogène ou le méthyle, sous réserve que
1) lorsque Y et Y' représentent le chlore, R n'est pas l'hydrogène, un alkyle en $C_1$-$C_3$, le n-butyle, le tert-butyle, l'allyle, le 2-méthoxyéthyle, le 2-éthoxyéthyle, le $\beta$-chloroéthyle, le méthallyle ou un alkyle en $C_5$ substitué par le reste $COOC_4H_9$ tert.; ou
2) lorsque Y et Y' représentent le brome, R n'est pas l'hydrogène, l'éthyle, le $\beta$-chloroéthyle, le $\beta$-méthoxyéthyle ou le $\beta$-éthoxyéthyle; ou
3) lorsque Y et Y' représentent l'iode, R n'est pas l'hydrogène ou l'éthyle; ou
4) lorsque Y et Y' représentent le fluor, R n'est pas l'hydrogène,

caractérisé en ce que l'on transforme l'acide 2,6-dichloroisonicotinique, l'acide 2,6-dibromoisonicotinique, l'acide 2,6-diiodoisonicotinique, l'acide difluoroisonicotinique ou leurs dérivés en esters.

**12.** Procédé pour la préparation des composés de formule I"

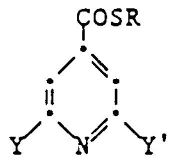

(I")

dans laquelle

Y et Y' représentent un halogène et

R représente l'hydrogène, un alkyle en $C_1$-$C_6$,un alkyle en $C_1$-$C_6$ interrompu par un atome d'oxygène ou par un atome de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou par le méthyle, sous réserve que, lorsque Y et Y' représentent le chlore, R n'est pas le méthyle ou le n-butyle,

caractérisé en ce que l'on transforme l'acide 2,6-dichloroisonicotinique, l'acide 2,6-dibromoisonicotinique, l'acide 2,6-diiodoisonicotinique, l'acide difluoroisonicotinique ou leurs dérivés en thioesters.

**13.** Procédé pour la préparation des composés de formule I'''

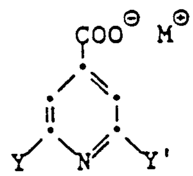

(I''')

dans laquelle

Y et Y' représentent un halogène et $M^{\oplus}$ représente un équivalent normal d'un cation, sous réserve que lorsque Y et Y' représentent le chlore, le brome ou l'iode, $M^{\oplus}$ n'est pas $Na^{\oplus}$, $K^{\oplus}$, 1/2 $Ca^{\oplus}$ ou $NH_4^{\oplus}$ ou, en outre, lorsque Y et Y' sont le chlore, $M^{\oplus}$ ne représente pas 1/2 $Ca^{\oplus}$.

**14.** Procédé pour la préparation des composés de formule $I^{IV}$

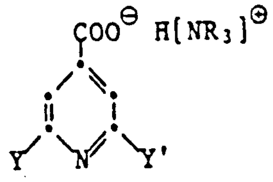

($I^{IV}$)

dans laquelle

Y et Y' représentent un halogène, $[NR_3]$ une alkylamine ayant 1 à 3 groupes alkyles en $C_1$-$C_6$ ou une alkylamine interrompue par un ou plusieurs atomes d'oxygène ayant 1 à 3 groupes alkyles en $C_1$-$C_6$ ou également les alkylamines cycliques suivantes :

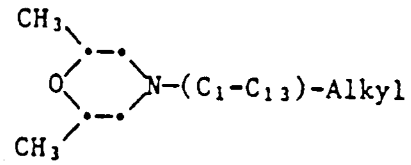 ; 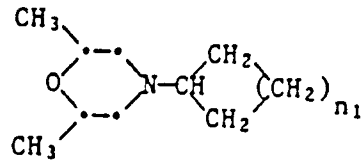 ,

où $n_1$ = 1-8

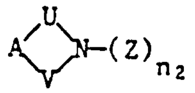 ,

où A représente l'oxygène ou le groupe méthylène, U et V un alkylène en $C_1$-$C_3$ ou un alkyle en $C_1$-$C_3$ alkylène, Z un alkylène en $C_1$-$C_4$, Ph un phényle non substitué ou substitué par un alkyle en $C_1$-$C_4$ et $n_2$ est égal à 0 ou 1, les énantiomères des structures chirales des alkylamines cycliques étant englobés,
caractérisé en ce que l'on transforme l'acide 2,6-dichloroisonicotinique, l'acide 2,6-dibromoisonicotinique, l'acide 2,6-diiodoisonicotinique et l'acide difluoroisonicotinique en sels d'amines.

**15.** Procédé pour la préparation de l'acide 2,6-dibromoisonicotinique ou de ses dérivés tels que les halogénures d'acide ou les anhydrides d'acide par transhalogénation de l'acide 2,6-dichloro-isonicotinique ou des dérivés correspondants avec l'acide bromhydrique gazeux dans un solvant organique à des températures de 20-150 ° C sous une pression de $1\text{-}100 \times 10^5$ Pa.

**16.** Agent pour prévenir et empêcher l'infestation des plantes par les microorganismes phytopathogènes, caractérisé en ce qu'il contient, à titre de substance active, au moins un composé de formule I selon la revendication 1.

**17.** Agent selon la revendication 16, caractérisé en ce qu'il contient, à titre de substance active, au moins un composé selon les revendications 2 à 6.

**18.** Agent pour prévenir et empêcher l'infestation des plantes par les microorganismes phytopathogènes, caractérisé en ce qu'il contient, à titre de substance active, au moins un composé de formule I'

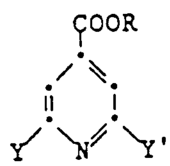

(I')

dans laquelle

Y et Y' représentent un halogène et

R représente l'hydrogène, un alkyle en $C_1$-$C_6$, un alkyle en $C_1$-$C_6$ interrompu par un atome d'oxygène ou de soufre, un alkyle en $C_1$-$C_6$ substitué par un halogène, par un cyano ou par le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou par un alcynyle en $C_3$-$C_5$ substitué par un halogène, un cycloalkyle en $C_3$-$C_6$ ou un cycloalkyle en $C_3$-$C_6$ substitué par un halogène ou le méthyle, sous réserve que

1) lorsque Y et Y' représentent le chlore, R n'est pas l'hydrogène, un alkyle en $C_1$-$C_3$, le n-butyle, le tert-butyle, l'allyle, le 2-méthoxyéthyle, le 2-éthoxyéthyle, le $\beta$-chloroéthyle, le méthallyle ou un alkyle en $C_5$ substitué par le reste $COOC_4H_9$ tert.; ou

2) lorsque Y et Y' représentent le brome, R n'est pas l'hydrogène, l'éthyle, le $\beta$-chloroéthyle, le $\beta$-méthoxyéthyle ou le $\beta$-éthoxyéthyle; ou

3) lorsque Y et Y' représentent l'iode, R n'est pas l'hydrogène ou l'éthyle; ou

4) lorsque Y et Y' représentent le fluor, R n'est pas l'hydrogène.

**19.** Agent pour prévenir et empêcher l'infestation des plantes par les microorganismes phytopathogènes, caractérisé en ce qu'il contient, à titre de substance active, au moins un composé de formule I"

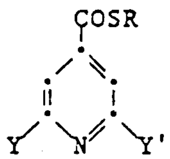

(I")

dans laquelle

Y et Y' représentent un halogène et

R représente l'hydrogène, un alkyle en $C_1$-$C_6$,un alkyle en $C_1$-$C_6$ interrompu par un atome d'oxygène ou par un atome de soufre, un alkyle en $C_1$-$C_6$ substitué par un halogène, le cyano ou le reste COO alkyle en $C_1$-$C_6$, un alcényle en $C_3$-$C_5$ ou un alcényle en $C_3$-$C_5$ substitué par un halogène, un alcynyle en $C_3$-$C_5$ ou un alcynyle en $C_3$-$C_5$ substitué par un halogène, un cycloalkyle en $C_3$-$C_6$ ou un cycloalkyle en $C_3$-$C_6$ substitué par un halogène ou par le méthyle, sous réserve que, lorsque Y et Y' représentent le chlore, R n'est pas le méthyle ou le n-butyle.

**20.** Agent pour prévenir et empêcher l'infestation des plantes par les microorganismes phytopathogènes, caractérisé en ce qu'il contient, à titre de substance active, au moins un composé de formule I'''

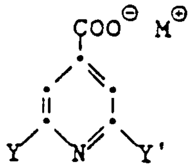

(I''')

dans laquelle

Y et Y' représentent un halogène et $M^{\oplus}$ représente un équivalent normal d'un cation, sous réserve que lorsque Y et Y' représentent le chlore, le brome ou l'iode, $M^{\oplus}$ n'est pas $Na^{\oplus}$, $K^{\oplus}$, 1/2 $Ca^{\oplus}$ ou $NH_4^{\oplus}$ ou, en outre, lorsque Y et Y' sont le chlore, $M^{\oplus}$ ne représente pas 1/2 $Ca^{\oplus}$.

**21.** Agent pour prévenir et empêcher l'infestation des plantes par les microorganismes phytopathogènes, caractérisé en ce qu'il contient, à titre de substance active, au moins un composé de formule $I^{IV}$

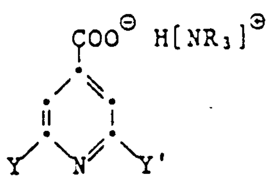

($I^{IV}$)

dans laquelle

Y et Y' représentent un halogène, $[NR_3]$ une alkylamine ayant 1 à 3 groupes alkyles en $C_1$-$C_6$ ou une alkylamine interrompue par un ou plusieurs atomes d'oxygène ayant 1 à 3 groupes alkyles en $C_1$-$C_6$ ou également les alkylamines cycliques suivantes :

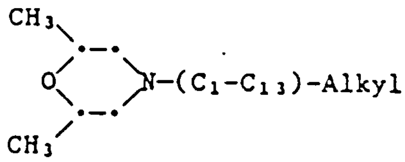 ; 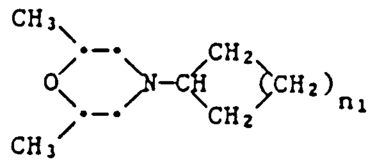 ,

où $n_1$ = 1-8

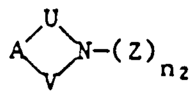 ,

où A représente l'oxygène ou le groupe méthylène, U et V un alkylène en $C_1$-$C_3$ ou un alkyle en $C_1$-$C_3$ alkylène, Z un alkylène en $C_1$-$C_4$, Ph un phényle non substitué ou substitué par un alkyle en $C_1$-$C_4$ et $n_2$ est égal à 0 ou 1, les énantiomères des structures chirales des alkylamines cycliques étant englobés.

**22.** Agent pour prévenir et empêcher l'infestation des plantes par des microorganismes phytopathogènes, caractérisé en ce qu'il contient, à titre de substance active, au moins un composé du groupe
sel de l'acide 2,6-dichloro-isonicotinique avec la N-[3-(4-tert-butylphényl)-2-méthyl-n-prop-1-yl]-2,6-diméthylmorpholine (comp. 4.8);
sel de l'acide 2,6-dichloro-isonicotinique avec la N-[3-(4-tert-butylphényl)-2-méthyl-n-prop-1-yl]-pipéridine (comp. 4.9);
ester propargylique de l'acide 2,6-dichloro-isonicotinique (comp. 1.29);
ester cyclohexylique de l'acide 2,6-dichloro-isonicotinique (comp. 1.25);
ester méthylique de l'acide 2,6-difluoro-isonicotinique (comp. 1.36).

**23.** Agent selon la revendication 16, caractérisé en ce qu'il contient, à titre de substance active, au moins un composé du groupe
acide 2,6-dichloro-isonicotinique (comp. 1.1);
ester méthylique de l'acide 2,6-dichloro-isonicotinique (comp. 1.2);
ester éthylique de l'acide 2,6-dichloro-isonicotinique (comp. 1.3);
acide 2,6-dibromo-isonicotinique (comp. 1.10).

**24.** Agent selon les revendications 16 à 23, caractérisé en ce qu'il contient 0,1 à 99 % d'une substance active de formule I, 99,9 à 1 % d'un additif solide ou liquide et 0 à 25 % d'un agent de surface.

**25.** Agent selon la revendication 24, caractérisé en ce qu'il contient 0,1 à 95 % d'une substance active de formule I, 99,8 à 5 % d'un additif solide ou liquide et 0,1 à 25 % d'un agent de surface.

**26.** Procédé selon la revendication 1, caractérisé en ce que l'on utilise un composé du groupe :
sel de l'acide 2,6-dichloro-isonicotinique avec la N-[3-(4-tert-butylphényl)-2-méthyl-n-prop-1-yl]-2,6-diméthylmorpholine (comp. 4.8); sel de l'acide 2,6-dichloro-isonicotinique avec la N-[3-(4-tert-butylphényl)-2-méthyl-n-prop-1-yl]-pipéridine (comp. 4.9); ester propargylique de l'acide 2,6-dichloro-isonicotinique (comp. 1.29); ester cyclohexylique de l'acide 2,6-dichloro-isonicotinique (comp. 1.25); ester méthylique de l'acide 2,6-difluoro-isonicotinique (comp. 1.36).

**27.** Utilisation des composés de formule I selon la revendication 1 pour prévenir et empêcher l'infestation des plantes par des microorganismes phytopathogènes.